# EUROPEAN PATENT APPLICATION

(11) **EP 2 332 925 A1**
(43) Date of publication of application: **15.06.2011**
(21) Application number: 09809876.7
(22) Date of filing: 24.08.2009
(51) Int. Cl.: C07D 251/10, A61K 8/43, A61K 8/49, A61K 31/155, A61K 31/53, A61P 31/02, A61P 31/04, C07C 279/26

(54) **NOVEL DIHYDROTRIAZINE DERIVATIVE**

(30) Priority: 25.08.2008 JP 2008215765
(71) Applicant: Hamari Chemicals, Ltd., Osaka-shi, Osaka 533-0024 (JP)
(72) Inventor: MAEDA, Shirou, Nagaokakyo-shi Kyoto 617-0857 (JP); MAEDA, Akihisa, Osaka-shi Osaka 544-0015 (JP)
(74) Representative: Duckworth, Timothy John
(86) International application number: PCT/JP2009/064739
(87) International publication number: WO 2010/024225

(57) **Abstract**

Provided is a compound represented by the following formula (1) 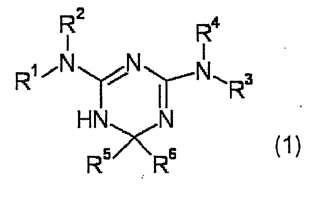 wherein
R¹ is a hydrogen atom, an optionally substituted alkyl group and the like, R² is a hydrogen atom or an optionally substituted alkyl group,
or R¹ and R² optionally form, together with the adjacent nitrogen atom, an optionally substituted nitrogen-containing heterocyclic group,
R⁴ is a hydrogen atom or an optionally substituted alkyl group,
R³ is an optionally substituted alkyl group, and
R⁵ and R⁶ are a hydrogen atom or a methyl group,
excluding a compound wherein both R² and R⁴ are hydrogen atoms and a compound wherein both R¹ and R⁴ are hydrogen atoms, or a tautomer thereof or a salt thereof useful as a bactericidal/disinfectant agent.

## Description

### Technical Field

The present invention relates to a novel dihydrotriazine derivative and a novel bactericidal/disinfectant agent comprising the derivative as an active ingredient.

### Background Art

Many infectious diseases have been overcome as a result of the development of various bactericides/disinfectants, antibiotics, and synthetic antibacterial agents, which has considerably extended the average life span of human beings.
On the other hand, however, a number of bacteria resistant to these medicaments, for example, methicillin-resistant *Staphylococcus aureus* (MRSA), vancomycin resistant enterococci (VRE), multidrug resistant *Pseudomonas aeruginosa* and the like, which are untreatable with conventional medicaments, have appeared to frequently cause opportunistic infection and hospital infection, thus raising a serious social problem.

Particularly, in the field of bactericidal/disinfectant agents, medicaments developed half a century ago, such as chlorhexidine gluconate and the like, are still widely used at present, and novel compounds have not been developed thereafter. Therefore, the development of a novel bactericidal/disinfectant agent showing an antibacterial activity at a low concentration by a contact for a short time with gram-positive resistant bacteria such as MRSA, VRE and the like, and gram negative bacteria such as *Pseudomonas aeruginosa* and the like, which replaces the existing bactericidal/disinfectant agents, has been desired. Since the finding of the effectiveness of 4,6-diamino-1-(p-chlorophenyl)-1,2-dihydro-2,2-dimethyl-s-triazine (Cycloguanil), which is an active metabolite of Proguanil (antimalarial agent), against malaria parasite 50 years or so ago (non-patent documents 1 and 2), diaminodihydrotriazine compounds have been reported in patent documents and non-patent documents relating to various antibacterial or antiparasitic actions.

For example, antivitamin activity and antimalarial activity of 4,6-diamino-2,2-dimethyl-s-triazine derivative and the like are disclosed (non-patent document 3). 4,6-Diamino-1,2-dihydro-2,2-dimethyl-1-phenyl-s-triazine and the like are disclosed in relation to antivitamin, antimalarial, anticancer or anticoccidial activity (non-patent document 4). Moreover, use of 1-(3-phenylpropyl)-2,4-diamino-6,6-dimethyl-1,6-dihydro-1,3,5-triazine and the like as insecticide is disclosed (patent document 1). The growth inhibitory action of 4,6-diamino-1,2-dihydro-1-phenyl-s-triazine and the like on *Pneumocystis carinii* is disclosed (patent document 2). Use of 1-p-chlorophenyl-4,6-diamino-1,2-dihydro-1,3,5-triazine and the like as anthelmintics (antimalarial agent and the like) is disclosed (patent document 3). As a compound further having an antibacterial activity besides an antimalarial action, 4,6-diamino-1,2-dihydro-1,3,5-triazine derivatives are disclosed (patent documents 4 and 5).

In patent document 6, Example 5, a compound having a herbicidal action, which is represented by the following formula, is disclosed.

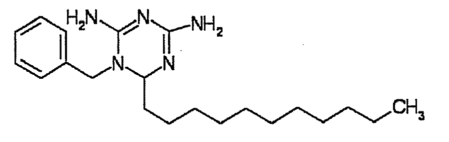

In patent document 7, Example 3, a compound having a herbicidal action, which is represented by the following formula, is disclosed.

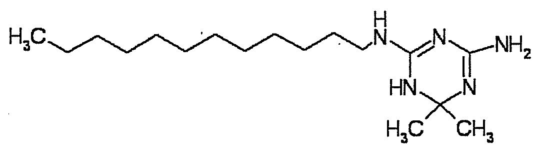

In non-patent document 5, a compound represented by the following formula is disclosed as a dihydrofolate reductase inhibitor (anthelmintic (antimalarial agent)).

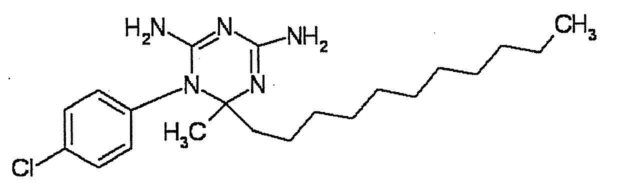

In patent document 8, an antibacterial agent characteristically containing a compound represented by the following formula or a salt thereof as an active ingredient is disclosed.

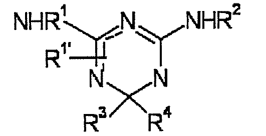

wherein
R¹ represents (i) a hydrogen atom, (ii) a phenyl group or a phenylalkyl group, each of which is optionally substituted, (iii) a naphthyl group or a naphthylalkyl group, each of which is optionally substituted, (iv) a heterocyclic group, a heterocyclic alkyl group or a heterocyclic aminoalkyl group, each of which is optionally substituted, (v) an optionally substituted alkyl group of 1 to 16 carbon atoms, or (vi) a cycloalkyl group or a cycloalkyl-alkyl group, each of which is optionally substituted;
(a) when R¹ is a hydrogen atom, R^{1'} represents (i) a phenyl group or a phenylalkyl group, each of which is optionally substituted, (ii) a naphthyl group or a naphthylalkyl group, each of which is optionally substituted, (iii) a heterocyclic group, a heterocyclic alkyl group or a heterocyclic aminoalkyl group, each of which is optionally substituted, (iv) an optionally substituted alkyl group of 1 to 16 carbon atoms, or (v) a cycloalkyl group or a cycloalkyl-alkyl group, each of which is substituted, said groups (i) to (v) being substituted at position 1 of the dihydrotriazine ring, or (b) when R¹ is other than a hydrogen atom, R^{1'} represents a hydrogen atom attached to the nitrogen atom at position 1 or 3 of the dihydrotriazine ring;
R² represents a hydrogen atom or an optionally substituted alkyl group of 1 to 16 carbon atoms;
R³ and R⁴ represent that R³ is a hydrogen atom or an optionally substituted alkyl group of 1 to 3 carbon atoms, and R⁴ is a hydrogen atom or an optionally substituted alkyl group of 1 to 16 carbon atoms, or R³ and R⁴ are taken together with the adjacent carbon atom to form a spirocycloalkane group or an alkyl spirocycloalkane group; and
the dashed line indicates that the position of a double bond is either between 1 and 2 or between 2 and 3.

### [Document List]

### [patent documents]

patent document 1: US 5,565,451 A
patent document 2: EP 0504290 B1
patent document 3: WO 01/53276 A1
patent document 4: US 3,682,912 A
patent document 5: US 3,723,429 A
patent document 6: US 3,287,365 A
patent document 7: US 3,287,366 A
patent document 8: WO 2004/054989 A1

### [non-patent documents]

non-patent document 1: Journal of Pharmacology, 1947, Vol. 2, p. 161-168
non-patent document 2: British H. C. Carrington et al., Nature, 1951, Vol. 168, p. 1080
non-patent document 3: E. J. Modest et al., Journal of the American Chemical Society, 1952, Vol. 74, p. 855-856
non-patent document 4: E. J. Modest et al., Journal of Organic Chemistry, 1956, Vol. 21, p. 1-13, p. 14-20
non-patent document 5: Andre Rosowsky et al., Antimicrobial Agents and Chemotherapy, 1995, Vol. 39, p. 79-86

### [SUMMARY OF THE INVENTION]

### Problems to be Solved by the Invention

It is an object of the present invention to provide a novel bactericidal/disinfectant agent comprising a dihydrotriazine compound or a pharmacologically acceptable salt thereof as an active ingredient.

### Means of Solving the Problems

The present inventors have conducted intensive studies in an attempt to solve the aforementioned problems, created a novel triazine derivative, and examined the physiological activity thereof and found that a dihydrotriazine compound represented by the following formula has a strong growth inhibitory effect and an antibacterial effect achievable in a short time on a wide spectrum of gram positive bacteria and gram negative bacteria, which resulted in the completion of the present invention. Accordingly, the present invention provides the following.
[1] A compound represented by the formula (1)

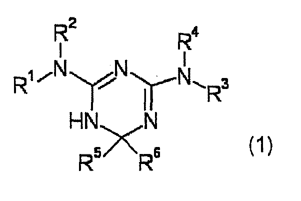

wherein
R¹ is a hydrogen atom, an optionally substituted alkyl group, an optionally substituted cycloalkyl group, an optionally substituted aryl group, an optionally substituted aralkyl group or an optionally substituted heterocyclic group,
R² is a hydrogen atom or an optionally substituted alkyl group, or R¹ and R² optionally form, together with the adjacent nitrogen atom, an optionally substituted nitrogen-containing heterocyclic group,
R⁴ is a hydrogen atom or an optionally substituted alkyl group, R³ is an optionally substituted alkyl group, and
R⁵ and R⁶ are the same or different and each is a hydrogen atom or a methyl group,
excluding a compound wherein both R² and R⁴ are hydrogen atoms and a compound wherein both R¹ and R⁴ are hydrogen atoms, or a tautomer thereof or a salt thereof.
[2] The compound according to the above-mentioned [1], wherein
   R¹ is a hydrogen atom, an optionally substituted alkyl group, an optionally substituted cycloalkyl group, an optionally substituted aryl group, an optionally substituted aralkyl group or an optionally substituted heterocyclic group,
   R² and R⁴ are the same or different and each is a hydrogen atom or an optionally substituted alkyl group,
   R³ is an optionally substituted alkyl group, and
   R⁵ and R⁶ are the same or different and each is a hydrogen atom or a methyl group,
   excluding a compound wherein both R² and R⁴ are hydrogen atoms and a compound wherein both R¹ and R⁴ are hydrogen atoms, or a tautomer thereof or a salt thereof.
[3] A compound represented by the formula (2)

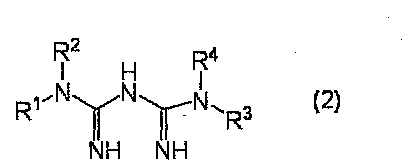

wherein
R¹ is a hydrogen atom, an optionally substituted alkyl group, an optionally substituted cycloalkyl group, an optionally substituted aryl group, an optionally substituted aralkyl group or an optionally substituted heterocyclic group,
R² is a hydrogen atom or an optionally substituted alkyl group, or R¹ and R² optionally form, together with the adjacent nitrogen atom, an optionally substituted nitrogen-containing heterocyclic group,
R⁴ is a hydrogen atom or an optionally substituted alkyl group, and
R³ is an optionally substituted alkyl group,
excluding a compound wherein both R² and R⁴ are hydrogen atoms and a compound wherein both R¹ and R⁴ are hydrogen atoms, or a tautomer thereof or a salt thereof.
[4] The compound according to the above-mentioned [3], wherein
   R¹ is a hydrogen atom, an optionally substituted alkyl group, an optionally substituted cycloalkyl group, an optionally substituted aryl group, an optionally substituted aralkyl group or an optionally substituted heterocyclic group,
   R² and R⁴ are the same or different and each is a hydrogen atom or an optionally substituted alkyl group, and
   R³ is an optionally substituted alkyl group,
   excluding a compound wherein both R² and R⁴ are hydrogen atoms and a compound wherein both R¹ and R⁴ are hydrogen atoms, or a tautomer thereof or a salt thereof.
[5] The compound according to any of the above-mentioned [1] to [4],
   wherein
   R¹ is an optionally substituted aralkyl group, or a tautomer thereof or a salt thereof.
[6] The compound according to any of the above-mentioned [1] to [5],
   wherein
   R² and R⁴ are the same or different and each is a hydrogen atom or a methyl group, excluding a compound wherein both R² and R⁴ are hydrogen atoms and a compound wherein both R¹ and R⁴ are hydrogen atoms, or a tautomer thereof or a salt thereof.
[7] The compound according to the above-mentioned [1] or [3], wherein
   R¹ and R² form, together with the adjacent nitrogen atom, an optionally substituted nitrogen-containing heterocyclic group, or a tautomer thereof or a salt thereof.
[8] The compound according to any of the above-mentioned [1] to [4],
   wherein
   R¹ and R² are each a hydrogen atom,
   excluding a compound wherein R⁴ is a hydrogen atom, or a tautomer thereof or a salt thereof.
[9] A method of producing a compound represented by the formula (1)

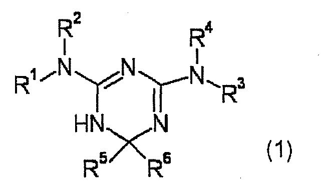

wherein each symbol is as defined in the above-mentioned [1], excluding a compound wherein both R² and R⁴ are hydrogen atoms and a compound wherein both R¹ and R⁴ are hydrogen atoms, or a tautomer thereof or a salt thereof, comprising reacting a compound represented by the formula (2)

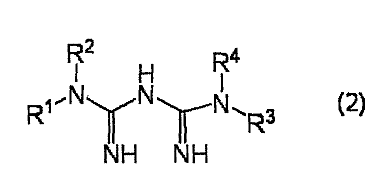

wherein each symbol is as defined in the above-mentioned [1], excluding a compound wherein both R² and R⁴ are hydrogen atoms and a compound wherein both R¹ and R⁴ are hydrogen atoms, or a tautomer thereof or a salt thereof, with
a compound represented by the formula (3)

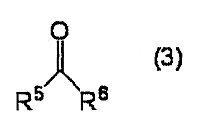

wherein each symbol is as defined in the above-mentioned [1].
[10] A bactericidal/disinfectant agent comprising a compound according to any of the above-mentioned [1] to [8], or a tautomer thereof or a salt thereof as an active ingredient.
[11] An antiseptic/preservative agent for cosmetics comprising a compound according to any of the above-mentioned [1] to [8], or a tautomer thereof or a salt thereof as an active ingredient.

### Effect of the Invention

A novel bactericidal/disinfectant agent, and an antiseptic/preservative agent for cosmetics, comprising a dihydrotriazine derivative in the present invention as an active ingredient show a strong antibacterial effect on a wide spectrum of gram positive bacteria and gram negative bacteria even by a treatment for a short time.

### [Description of Embodiments]

The present invention is explained in detail in the following.
As the "alkyl group" of the "optionally substituted alkyl group", a linear or branched chain alkyl group can be mentioned, and examples thereof include C₁₋₁₆ alkyl groups (e.g., methyl, ethyl, n-propyl, isopropyl, n-butyl, isobutyl, sec-butyl, tert-butyl, n-hexyl, n-heptyl, n-octyl, tert-octyl, n-nonyl, n-decyl, n-undecyl, n-dodecyl, n-tridecyl, n-tetradecyl, n-pentadecyl, n-hexadecyl and the like).

As the substituents that the alkyl group of the "optionally substituted alkyl group" optionally has, substituents selected from
(i) a halogen atom (e.g., fluorine atom, chlorine atom, bromine atom, iodine atom),
(ii) a cyano group,
(iii) a hydroxyl group,
(iv) a nitro group,
(v) a formyl group,
(vi) a thiol group,
(vii) a C₃₋₆ cycloalkyl group (e.g., cyclopropyl, cyclobutyl, cyclopentyl, cyclohexyl etc.),
(viii) a C₆₋₁₀ aryloxy group (e.g., phenyloxy, naphthyloxy etc.),
(ix) a C₁₋₆ alkoxy group (e.g., methoxy, ethoxy, n-propoxy, isopropoxy, n-butoxy, isobutoxy etc.),
(x) a C₁₋₆ haloalkoxy group (e.g., trifluoromethoxy etc.),
(xi) a C₃₋₆ cycloalkyloxy group (e.g., cyclopropyloxy, cyclobutyloxy, cyclopentyloxy, cyclohexyloxy etc.),
(xii) a C₁₋₇ alkanoyl group (e.g., formyl, acetyl, propionyl, butyryl, isobutyryl, pentanoyl, hexanoyl etc.),
(xiii) a carboxyl group,
(xiv) a carbamoyl group,
(xv) a C₁₋₆ alkoxy-carbonyl group (e.g., methoxycarbonyl, ethoxycarbonyl, n-propoxycarbonyl, isopropoxycarbonyl, n-propoxycarbonyl, isobutoxycarbonyl etc.),
(xvi) a C₁₋₆ haloalkoxy-carbonyl group (e.g., chloromethoxycarbonyl, bromomethoxycarbonyl, (1-chloro)ethoxycarbonyl etc.),
(xvii) a C₆₋₁₄ aryloxy-carbonyl group (e.g., phenyloxycarbonyl, naphthyloxycarbonyl etc.),
(xviii) a C₃₋₆ cycloalkyloxy-carbonyl group (e.g., cyclopropoxycarbonyl, cyclopentyloxycarbonyl etc.),
(xix) an amino group,
(xx) a C₁₋₆ alkylamino group (e.g., methylamino, ethylamino, n-propylamino, isopropylamino, sec-butylamino, n-pentylamino etc.),
(xxi) a C₁₋₆ haloalkylamino group (e.g., trifluoromethylamino etc.),
(xxii) a di C₁₋₆ alkylamino group (e.g., dimethylamino, diethylamino, methylethylamino etc.),
(xxiii) a C₁₋₇ alkanoyl-amino group (e.g., substituent wherein the above-mentioned C₁₋₇ alkanoyl group bonded with amino group etc.),
(xxiv) a C₁₋₆ alkylamino-carbonyl group (e.g., dimethylaminocarbonyl, diethylaminocarbonyl, methylethylaminocarbonyl etc.),
(xxv) a mercapto group,
(xxvi) a sulfonic acid group,
(xxvii) a sulfonamide group,
(xxviii) a C₁₋₆ alkylthio group (e.g., methylthio, ethylthio, n-propylthio, isopropylthio, sec-butylthio, n-pentylthio etc.),
(xxix) a C₁₋₆ haloalkylthio group (e.g., trifluoromethylthio etc.),
(xxx) a C₁₋₆ alkylsulfonyl group (e.g., methylsulfonyl, ethylsulfonyl, n-propylsulfonyl, isopropylsulfonyl, n-butylsulfonyl, isobutylsulfonyl, sec-butylsulfonyl, tert-butylsulfonyl, n-pentylsulfonyl, sec-pentylsulfonyl, isopentylsulfonyl, neopentylsulfonyl, n-hexylsulfonyl or isohexylsulfonyl etc.),
(xxxi) a C₁₋₆ haloalkylsulfonyl group (e.g., chloromethylsulfonyl, trifluoromethylsulfonyl etc.),
(xxxii) a C₁₋₆ alkylsulfonyloxy group,
(xxxiii) a C₁₋₆ haloalkylsulfonyloxy group (e.g., chloromethylsulfonyloxy, trifluoromethylsulfonyloxy etc.),
(xxxiv) a C₁₋₆ alkylsulfonylamino group (e.g., substituent wherein the above-mentioned C₁₋₆ alkylsulfonyl group is bonded with amino group etc.),
(xxxv) a C₁₋₆ haloalkylsulfonylamino group (e.g., substituent wherein the above-mentioned C₁₋₆ haloalkylsulfonyl group is bonded with amino group etc.),
(xxxvi) a 5- to 14-membered nonaromatic heterocyclic group containing, besides carbon atoms, 1 to 4 hetero atoms selected from a nitrogen atom,'a sulfur atom and an oxygen atom (for example, pyrrolidinyl, tetrahydrofuryl, 2-oxotetrahydrofuryl, tetrahydrothienyl, piperidyl (e.g., 1-piperidyl), tetrahydropyranyl, 2-oxotetrahydropyranyl, morpholinyl, thiomorpholinyl, piperazinyl (e.g., 1-piperazinyl), azepanyl, 1,4-diazepanyl, oxazepanyl (e.g., 1,4-oxazepanyl), benzotriazolyl, 3,4-dihydro-2H-benzo[1,4]oxazin etc.), and optionally substituted by 1 to 3 substituents selected from a halogen atom (e.g., fluorine atom, chlorine atom, bromine atom, iodine atom) and a C₁₋₆ alkyl group (e.g., methyl, ethyl, propyl etc.),
(xxxvii) a 5- to 14-membered aromatic heterocyclic group containing, besides carbon atoms, 1 to 4 hetero atoms selected from a nitrogen atom, a sulfur atom and an oxygen atom (for example, furyl (e.g., 2-furyl), thienyl, pyrrolyl, oxazolyl, isoxazolyl, thiazolyl (e.g., 2-thiazolyl), isothiazolyl, imidazolyl, pyrazolyl, oxadiazolyl (e.g., 1,2,3-oxadiazolyl, 1,2,4-oxadiazolyl, 1,3,4-oxadiazolyl), furazanyl, thiadiazolyl (e.g., 1,2,3-thiadiazolyl, 1,2,4-thiadiazolyl, 1,3,4-thiadiazolyl), triazolyl (e.g., 1,2,3-triazolyl, 1,2,4-triazolyl), tetrazolyl, pyridyl (e.g., 2-pyridyl, 3-pyridyl, 4-pyridyl), pyridazinyl, pyrimidinyl, pyrazinyl, triazinyl (e.g., 1,3,5-triazinyl, 1,2,4-triazinyl), imidazopyridyl, indolyl, quinolyl (e.g., 2-quinolyl, 3-quinolyl, 4-quinolyl, 5-quinolyl, 8-quinolyl), isoquinolyl (e.g., 1-isoquinolyl, 3-isoquinolyl, 4-isoquinolyl, 5-isoquinolyl) etc.), and
optionally substituted by 1 to 3 substituents selected from a halogen atom (e.g., fluorine atom, chlorine atom, bromine atom, iodine atom) and a C₁₋₆ alkyl group (e.g., methyl, ethyl, propyl etc.) can be mentioned. Such substituents can be present at any chemically acceptable positions. The number of substituents is 1 to 6, preferably 1 to 3.

As the "optionally substituted cycloalkyl group", a C₃₋₆ cycloalkyl group (e.g., cyclopropyl, cyclobutyl, cyclopentyl, cyclohexyl) optionally having 1 to 6, preferably 1 to 3, substituents selected from
(a) substituent that the above-mentioned "optionally substituted alkyl group" optionally has,
(b) a C₁₋₆ alkyl group (e.g., methyl, ethyl, n-propyl, isopropyl, n-butyl, isobutyl, sec-butyl, tert-butyl, n-pentyl, sec-pentyl, isopentyl, neopentyl, n-hexyl, isohexyl etc.),
(c) a C₁₋₆ haloalkyl group (e.g., chloromethyl, bromomethyl, 1-chloroethyl, trifluoromethyl etc.), and
(d) a C₆₋₁₄ aryl group (e.g., phenyl, 1-naphthyl, 2-naphthyl etc.),
at substitutable position(s) can be mentioned.

As the "optionally substituted aryl group", a C₆₋₁₄ aryl group (e.g., phenyl, 1-naphthyl, 2-naphthyl etc.) optionally having 1 to 4, preferably 1 to 3, substituents that the cycloalkyl group of the above-mentioned "optionally substituted cycloalkyl group" optionally has can be mentioned.

As the "optionally substituted aralkyl group", a C₇₋₁₂ aralkyl group (e.g., benzyl, aminobenzyl, nitrobenzyl, 2-phenylethyl, 1-phenylethyl, 1-phenylpropyl, 2-phenylpropyl etc.) optionally having 1 to 6, preferably 1 to 3, substituents that the cycloalkyl group of the above-mentioned "optionally substituted cycloalkyl group" optionally has, at substitutable position(s), can be mentioned.

In the present specification, as the "optionally substituted heterocyclic group",
(a) a 5- to 14-membered nonaromatic heterocyclic group containing, besides carbon atoms, 1 to 4 hetero atoms selected from a nitrogen atom, a sulfur atom and an oxygen atom (e.g., pyrrolidinyl (e.g., 1-pyrrolidinyl), tetrahydrofuryl, 2-oxotetrahydrofuryl, tetrahydrothienyl, piperidyl (e.g., 1-piperidyl), tetrahydropyranyl, 2-oxotetrahydropyranyl, morpholinyl (e.g., 4-morpholinyl), thiomorpholinyl (e.g., 4-thiomorpholinyl), piperazinyl (e.g., 1-piperazinyl), azepanyl, 1,4-diazepanyl, oxazepanyl (e.g., 1,4-oxazepanyl), benzotriazolyl, 3,4-dihydro-2H-benzo[1,4]oxazin etc.), and optionally having 1 to 6, preferably 1 to 3, substituents that the cycloalkyl group of the above-mentioned "optionally substituted cycloalkyl group" optionally has, at a substitutable position, and
(b) a 5- to 14-membered aromatic heterocyclic group containing, besides carbon atoms, 1 to 4 hetero atoms selected from a nitrogen atom, a sulfur atom and an oxygen atom (e.g., furyl (e.g., 2-furyl), thienyl, pyrrolyl, oxazolyl, isoxazolyl, thiazolyl (e.g., 2-thiazolyl), isothiazolyl, imidazolyl, pyrazolyl, oxadiazolyl (e.g., 1,2,3-oxadiazolyl, 1,2,4-oxadiazolyl, 1,3,4-oxadiazolyl), furazanyl, thiadiazolyl (e.g., 1,2,3-thiadiazolyl, 1,2,4-thiadiazolyl, 1,3,4-thiadiazolyl), triazolyl (e.g., 1,2,3-triazolyl, 1,2,4-triazolyl), tetrazolyl, pyridyl (e.g., 2-pyridyl, 3-pyridyl, 4-pyridyl), pyridazinyl, pyrimidinyl, pyrazinyl, triazinyl (e.g., 1,3,5-triazinyl, 1,2,4-triazinyl), imidazopyridyl, indolyl, quinolyl (e.g., 2-quinolyl, 3-quinolyl, 4-quinolyl, 5-quinolyl, 8-quinolyl), isoquinolyl (e.g., 1-isoquinolyl, 3-isoquinolyl, 4-isoquinolyl, 5-isoquinolyl), and optionally having 1 to 6, preferably 1 to 3, substituents that the cycloalkyl group of the above-mentioned "optionally substituted cycloalkyl group" optionally has, at substitutable position(s). In addition, the "optionally substituted heterocyclic group" may be oxidized. For example, when the heterocyclic group contains a nitrogen atom and/or a sulfur atom, the nitrogen atom and/or the sulfur atom may be oxidized.

The "optionally substituted heterocyclic group" may be fused with a C₃₋₈ cycloalkyl ring (e.g., cyclopropane ring, cyclobutane ring, cyclopentane ring, cyclohexane ring, cycloheptane ring, cyclooctane ring) or fused with a C₆₋₁₄ aryl ring (e.g., benzene ring, naphthalene ring etc.), or may be bonded to a C₃₋₈ cycloalkyl ring (e.g., cyclopropane ring, cyclobutane ring, cyclopentane ring, cyclohexane ring, cycloheptane ring, cyclooctane ring), a condensed cycloalkyl ring (e.g., indane ring etc.) and the like to form a spiro ring.

In the present specification, as the "optionally substituted nitrogen-containing heterocyclic group", a 5- to 14-membered nonaromatic nitrogen-containing heterocyclic group containing at least one nitrogen atom and optionally containing, besides the nitrogen atom and carbon atoms, 1 to 4 hetero atoms selected from a nitrogen atom, a sulfur atom and an oxygen atom (e.g., 1-pyrrolidinyl, 1-piperidyl, 4-morpholinyl, 4-thiomorpholinyl, 1-piperazinyl, 1-azepanyl, 1,4-diazepan-1-yl, 1,4-oxazepan-4-yl), and optionally having 1 to 6, preferably 1 to 3, substituents that the cycloalkyl group of the above-mentioned "optionally substituted cycloalkyl group" optionally has, at a substitutable position, and the like can be mentioned. In addition, the "optionally substituted nitrogen-containing heterocyclic group" may be oxidized. For example, when the nitrogen-containing heterocyclic group contains a nitrogen atom and/or a sulfur atom, the nitrogen atom and/or the sulfur atom may be oxidized.
The "optionally substituted nitrogen-containing heterocyclic group" may be fused with a C₃₋₈ cycloalkyl ring (e.g., cyclopropane ring, cyclobutane ring, cyclopentane ring, cyclohexane ring, cycloheptane ring, cyclooctane ring) or a C₆₋₁₄ aryl ring (e.g., benzene ring, naphthalene ring etc.) or may be bonded to a C₃₋₈ cycloalkyl ring (e.g., cyclopropane ring, cyclobutane ring, cyclopentane ring, cyclohexane ring, cycloheptane ring, cyclooctane ring), a condensed cycloalkyl ring (e.g., indane ring etc.) and the like to form a spiro ring.

In compound (1) and compound (2),
R¹ is a hydrogen atom, an optionally substituted alkyl group, an optionally substituted cycloalkyl group, an optionally substituted aryl group, an optionally substituted aralkyl group or an optionally substituted heterocyclic group, and R² is a hydrogen atom or an optionally substituted alkyl group (provided that R² and R⁴, and R¹ and R⁴ are not hydrogen atoms at the same time). Alternatively, R¹ and R² optionally form, together with the adjacent nitrogen atom, an optionally substituted nitrogen-containing heterocyclic group.

The "optionally substituted alkyl group" for R¹ is preferably an optionally substituted C₁₋₁₆ alkyl group.

As the substituent of the "optionally substituted alkyl group" for R¹, a C₁₋₆ alkoxy group (e.g., methoxy group) is more preferable.

As the "optionally substituted cycloalkyl group" for R¹, an optionally substituted C₃₋₆ cycloalkyl group is preferable.

As the "optionally substituted aryl group" for R¹, an optionally substituted C₆₋₁₄ aryl group is preferable.

As the "optionally substituted aralkyl group" for R¹, an optionally substituted C₇₋₁₂ aralkyl group is preferable.

As the substituent of the optionally substituted aralkyl group for R¹,
(1) a C₁₋₁₆ alkyl group (preferably, C₁₋₆ alkyl group) optionally having 1 to 3 substituents selected from
   (i) a halogen atom(preferably, fluorine atom, chlorine atom);
   (ii) a hydroxyl group;
   (iii) a C₁₋₆ alkoxy group (preferably, methoxy group);
   (iv) a C₁₋₆ haloalkoxy group (preferably, trifluoromethoxy group); and
   (v) a C₃₋₆ cycloalkyl group (preferably, cyclohexyl group);
(2) a C₆₋₁₄ aryl group (preferably, phenyl group, naphthyl group) optionally having 1 to 3 substituents selected from
   (i) a halogen atom (preferably, fluorine atom, chlorine atom);
   (ii) a hydroxyl group;
   (iii) a C₁₋₆ alkyl group (preferably, methyl group, tert-butyl group);
   (iv) a C₁₋₆ haloalkyl group (preferably, trifluoromethyl group);
   (v) a C₁₋₆ alkoxy group (preferably, methoxy group);
   (vi) a C₁₋₆ haloalkoxy group (preferably, trifluoromethoxy group);
   (vii) a C₃₋₆ cycloalkyl group (preferably, cyclohexyl group); and
   (viii) a C₆₋₁₄ aryl group (preferably, phenyl group, naphthyl group);
(3) a C₇₋₁₂ aralkyl group (preferably benzyl group or 2-phenylethyl group) optionally having 1 to 3 substituents selected from
   (i) a halogen atom (preferably, fluorine atom, chlorine atom);
   (ii) a hydroxyl group;
   (iii) a C₁₋₈ alkyl group (preferably, methyl group, tert-butyl group);
   (iv) a C₁₋₈ haloalkyl group (preferably, trifluoromethyl group);
   (v) a C₁₋₆ alkoxy group (preferably, methoxy group);
   (vi) a C₁₋₆ haloalkoxy group (preferably, trifluoromethoxy group);
   (vii) a C₃₋₆ cycloalkyl group (preferably, cyclohexyl group); and
   (viii) a C₆₋₁₄ aryl group (preferably, phenyl group, naphthyl group);
(4) an amino group;
(5) a nitro group; and
(6) a halogen atom
are more preferable.

As the "optionally substituted alkyl group" for R², an optionally substituted C₁₋₃ alkyl group is preferable. As the C₁₋₃ alkyl group, methyl group, ethyl group, n-propyl group, isopropyl group and the like can be mentioned. As the substituent, a halogen atom (preferably, fluorine atom) and the like can be mentioned.
R² is more preferably a hydrogen atom, a methyl group, an ethyl group or a trifluoromethyl group, futher preferably a hydrogen atom, a methyl group or an ethyl group (provided that R² and R⁴ are not hydrogen atoms at the same time).

As the "optionally substituted nitrogen-containing heterocyclic group" formed by R¹ and R² together with the adjacent nitrogen atom, preferred are a 5- to 14-membered nonaromatic nitrogen-containing heterocyclic group containing at least one nitrogen atom and optionally containing, besides the nitrogen atom and carbon atoms, 1 to 4 hetero atoms selected from a nitrogen atom, a sulfur atom and an oxygen atom (e.g., 1-pyrrolidinyl, 1-piperidyl, 4-morpholinyl, 4-thiomorpholinyl, 1-piperazinyl, 1-azepanyl, 1,4-diazepan-1-yl, 1,4-oxazepan-4-yl) and a fused ring group (e.g., dihydroisoindol-2-yl) of the nonaromatic nitrogen-containing heterocyclic group and a C₆₋₁₄ aryl ring (e.g., benzene ring).

As the substituent of the "optionally substituted nitrogen-containing heterocyclic group" formed by R¹ and R² together with the adjacent nitrogen atom, a C₁₋₆ alkyl group (e.g., methyl group) and a C₆₋₁₄ aryl group (e.g., phenyl group) are more preferable.

R¹ is more preferably a hydrogen atom, a phenyl group, a benzyl group, a 4-chlorophenyl group, a 2,4-difluorophenyl group, a 2,3,4-trifluorophenyl group, a 4-tert-butylphenyl group, a 4-methoxyphenyl group, a 2-methoxy-4-tert-butylphenyl group, a 4-trifluoromethoxyphenyl group, a 4-hydroxylbenzyl group, a 3,4-dichlorobenzyl group, a 2,3,4-trichlorobenzyl group, a 4-methylbenzyl group, a 4-trifluoromethylbenzyl group, a 4-methoxybenzyl group, a 3,4-dimethoxybenzyl group, a 4-aminophenyl group, a 4-nitrophenyl group, a 2-phenylethyl group, a 2-(4-methoxyphenyl)ethyl group, an ethyl group, a 2-methoxyethyl group, an n-propyl group, an isopropyl group, an n-hexyl group, an n-heptyl group, an n-octyl group, an n-tetradecyl group, a cyclohexyl group, a cyclohexylmethyl group and the like (provided that R¹ and R⁴ are not hydrogen atoms at the same time),
R² is more preferably a hydrogen atom, a methyl group or an ethyl group (provided that R¹ and R⁴ are not hydrogen atoms at the same time) and
the nitrogen-containing heterocyclic group formed by R¹ and R² together with the adjacent nitrogen atom is more preferably a 4-morpholinyl group, a 4-thiomorpholinyl group, a 4-methylpiperazin-1-yl group, a 4-phenylpiperazin-1-yl group, a 1-piperidinyl group, a 1,2-dihydroisoindol-2-yl group or the like.

In compound (1) and compound (2),
R⁴ is a hydrogen atom or an optionally substituted alkyl group (provided that R² and R⁴, and R¹ and R⁴ are not hydrogen atoms at the same time). As the "optionally substituted alkyl group" for R⁴, an optionally substituted C₁₋₃ alkyl group is preferable. As the C₁₋₃ alkyl group, a methyl group, an ethyl group, an n-propyl group, an isopropyl group and the like can be mentioned. As the substituent, a halogen atom (preferably fluorine atom) and the like can be mentioned.

R⁴ is more preferably a hydrogen atom, a methyl group or a trifluoromethyl group, and more preferably a hydrogen atom or a methyl group (provided that R² and R⁴, and R¹ and R⁴ are not hydrogen atoms at the same time).

In compound (1) and compound (2),
R³ is an optionally substituted alkyl group. The "optionally substituted alkyl group" for R³ is preferably an optionally substituted C₁₋₁₆ alkyl group, more preferably a C₁₋₁₆ alkyl group (e.g., n-octyl, n-nonyl, n-decyl, n-undecyl, n-dodecyl, n-tridecyl, n-tetradecyl etc.).

In compound (1),
R⁵ and R⁶ are the same or different and each is a hydrogen atom or a methyl group. As R⁵ and R⁶, a methyl group is preferable.

As compound (1), a compound wherein
R¹ is
a hydrogen atom;
a C₁₋₁₆ alkyl group optionally having 1 to 3 substituents selected from a halogen atom, a hydroxyl group, a C₁₋₆ alkoxy group, a C₁₋₆ haloalkoxy group, and a C₃₋₆ cycloalkyl group;
a C₃₋₆ cycloalkyl group having 1 to 3 substituents selected from a halogen atom, a hydroxyl group, a C₁₋₆ alkoxy group, a C₁₋₆ haloalkoxy group, and a C₃₋₆ cycloalkyl group;
a C₆₋₁₄ aryl group optionally having 1 to 3 substituents selected from a halogen atom (preferably, fluorine atom, chlorine atom), a hydroxyl group, a C₁₋₆ alkyl group, a C₁₋₆ haloalkyl group, a C₁₋₆ alkoxy group, a C₁₋₆ haloalkoxy group, a C₃₋₆ cycloalkyl group, and a C₆₋₁₄ aryl group; or
a C₇₋₁₂ aralkyl group optionally having 1 to 3 substituents selected from a halogen atom, a hydroxyl group, an amino group, a C₁₋₆ alkyl group, a C₁₋₆ haloalkyl group, a C₁₋₆ alkoxy group, a C₁₋₆ haloalkoxy group, a C₃₋₆ cycloalkyl group, and a C₆₋₁₄ aryl group,
R² is a hydrogen atom or a C₁₋₃ alkyl group, or R¹ and R² form, together with the adjacent nitrogen atom, an optionally substituted nitrogen-containing heterocyclic group,
R⁴ is a hydrogen atom or a methyl group,
R³ is a C₁₋₁₆ alkyl group, and
R⁵ and R⁶ are the same or different and each is a hydrogen atom or a methyl group,
excluding a compound wherein both R² and R⁴ are hydrogen atoms and a compound wherein both R¹ and R⁴ are hydrogen atoms, is preferable, and

a compound wherein
R¹ is
a hydrogen atom;
a C₁₋₆ alkyl group (e.g., methyl group, ethyl group, n-propyl group) optionally having 1 to 3 C₁₋₆ alkoxy groups (e.g., methoxy group);
a C₃₋₆ cycloalkyl group (e.g., cyclohexyl group); or a C₇₋₁₂ aralkyl group (e.g., benzyl group, 2-phenylethyl group) optionally having 1 to 3 substituents selected from a halogen atom (e.g., chlorine atom), a C₁₋₆ alkyl group (e.g., methyl group), and an amino group,
R² is a hydrogen atom, a methyl group or an ethyl group, or
R¹ and R² form, together with the adjacent nitrogen atom, a nitrogen-containing heterocyclic group (e.g., 4-morpholinyl group, 4-thiomorpholinyl group, 4-methylpiperazin-1-yl group, 4-phenylpiperazin-1-yl group, 1-piperidinyl group, 1,2-dihydroisoindol-2-yl group) optionally having 1 to 3 substituents selected from a C₁₋₆ alkyl group (e.g., methyl group) and a C₆₋₁₄ aryl group (e.g., phenyl group),
R⁴ is a hydrogen atom or a methyl group,
R³ is a C1-16 alkyl group (e.g., n-octyl, n-nonyl, n-decyl, n-undecyl, n-dodecyl, n-tridecyl, n-tetradecyl), and
R⁵ and R⁶ are each a methyl group,
excluding a compound wherein both R² and R⁴ are hydrogen atoms and a compound wherein both R¹ and R⁴ are hydrogen atoms, is more preferable.

As compound (1), the compounds described in Examples 1 to 26 are more preferable.

As compound (2), a compound wherein
R¹ is
a hydrogen atom;
a C₁₋₁₆ alkyl group optionally having 1 to 3 substituents selected from a halogen atom, a hydroxyl group, a C₁₋₆ alkoxy group, a C₁₋₆ haloalkoxy group, and a C₃₋₆ cycloalkyl group;
a C₃₋₆ cycloalkyl group optionally having 1 to 3 substituents selected from a halogen atom, a hydroxyl group, a C₁₋₆ alkoxy group, a C₁₋₆ haloalkoxy group, and a C₃₋₆ cycloalkyl group;
a C₆₋₁₄ aryl group optionally having 1 to 3 substituents selected from a halogen atom (preferably, fluorine atom, chlorine atom), a hydroxyl group, a C₁₋₆ alkyl group, a C₁₋₆ haloalkyl group, a C₁₋₆ alkoxy group, a C₁₋₆ haloalkoxy group, a C₃₋₆ cycloalkyl group and a C₆₋₁₄ aryl group; or
a C₇₋₁₂ aralkyl group optionally having 1 to 3 substituents selected from a halogen atom, a hydroxyl group, a nitro group, a C₁₋₆ alkyl group, a C₁₋₆ haloalkyl group, a C₁₋₆ alkoxy group, a C₁₋₆ haloalkoxy group, a C₃₋₆ cycloalkyl group, and a C₆₋₁₄ aryl group,
R² is a hydrogen atom or a C₁₋₃ alkyl group, or
R¹ and R² form, together with the adjacent nitrogen atom, an optionally substituted nitrogen-containing heterocyclic group,
R⁴ is a hydrogen atom or a methyl group, and
R³ is a C₁₋₁₆ alkyl group,
excluding a compound wherein both R² and R⁴ are hydrogen atoms and a compound wherein both R² and R⁴ are hydrogen atoms is preferable, and

a compound wherein
R¹ is
a hydrogen atom;
a C₁₋₆ alkyl group (e.g., methyl group, ethyl group, n-propyl group) optionally having 1 to 3 C₁₋₆ alkoxy groups (e.g., methoxy group);
a C₃₋₆ cycloalkyl group (e.g., cyclohexyl group); or a C₇₋₁₂ aralkyl group (e.g., benzyl group, 2-phenylethyl group) optionally having 1 to 3 substituents selected from a halogen atom (e.g., chlorine atom), a C₁₋₆ alkyl group (e.g., methyl group) and a nitro group,
R² is a hydrogen atom, a methyl group or an ethyl group, or
R¹ and R² form, together with the adjacent nitrogen atom, a nitrogen-containing heterocyclic group (e.g., 4-morpholinyl group, 4-thiomorpholinyl group, 4-methylpiperazin-1-yl group, 4-phenylpiperazin-1-yl group, 1-piperidinyl group, 1,2-dihydroisoindol-2-yl group) optionally having 1 to 3 substituents selected from a C₁₋₆ alkyl group (e.g., methyl group) and a C₆₋₁₄ aryl group (e.g., phenyl group),
R⁴ is a hydrogen atom or a methyl group, and
R³ is a C1-16 alkyl group (e.g., n-octyl, n-nonyl, n-decyl, n-undecyl, n-dodecyl, n-tridecyl, n-tetradecyl), excluding a compound wherein both R² and R⁴ are hydrogen atoms and a compound wherein both R¹ and R⁴ are hydrogen atoms, is more preferable.

As compound (2), the compounds described in Examples 27 to 50 are more preferable.

The above-mentioned compounds (1) and (2) may form a salt. Examples of such salt include salts with organic acids such as formic acid, acetic acid, propionic acid, lactic acid, butyric acid, isobutyric acid, trifluoroacetic acid, malic acid, maleic acid, malonic acid, fumaric acid, succinic acid, succinic acid monoamide, glutamic acid, tartaric acid, oxalic acid, citric acid, glycolic acid, gluconic acid, ascorbic acid, benzoic acid, phthalic acid, salicylic acid, anthranilic acid, benzenesulfonic acid, p-toluenesulfonic acid, methanesulfonic acid and the like; and salts with inorganic acids such as hydrochloric acid, hydrobromic acid, sulfuric acid, phosphoric acid, nitric acid, carbonic acid, boric acid, carbonic acid and the like. The above-mentioned acid addition salts are produced by, for example, employing a salt formation method such as (a) direct mixing of the above-mentioned compound (1) or (2) and an acid, (b) mixing with one of them dissolved in a solvent or aqueous solvent, (c) mixing after adding the above-mentioned compound (1) or (2) and an acid into a solvent or aqueous solvent or blending compound (1) and esters such as ethyl acetate and the like or lactones such as gluconolactone and the like, and the like.

When the above-mentioned compound (1) and compound (2) have an acidic group such as a carboxyl group, a sulfonic acid group and the like, the above-mentioned compound (1) and compound (2) form a zwitter ion salt. The salt may be, for example, a base addition salt such as an alkali metal salt such as sodium salt, potassium salt and the like, an alkaline earth metal salt such as calcium salt, magnesium salt and the like, a salt with inorganic base such as aluminum salt, ammonium salt and the like; a salt with an organic base such as trimethylamine, triethylamine, pyridine, picoline, ethanolamine, diethanolamine, triethanolamine, dicyclohexylamine, N,N'-dibenzylethylenediamine and the like, and the like. A salt of the above-mentioned compound (1) and compound (2) may be, for example, a salt with basic amino acid such as arginine, lysine, ornithine and the like, or a salt with acidic amino acid such as aspartic acid and the like.

The salts of the above-mentioned compound (1) and compound (2) are preferably pharmacologically acceptable, more preferably acid addition salts, and further preferably gluconate, acetate, hydrochloride, hydrobromide, carbonate, methanesulfonate, malonate, oxalate and the like.

Compound (1) and compound (2) encompass solvate, for example, hydrate. In addition, compound (1) and compound (2) may be labeled with an isotope (e.g., ³H, ¹⁴C, ³⁵S, ¹²⁵I and the like) and the like. Compound (1) and compound (2) may also be deuterium exchanged compounds.

When compound (1) and compound (2) of the present invention has an asymmetric center, isomers such as enantiomer, diastereomer and the like can be present. Such isomers and mixtures thereof are all encompassed in the present invention. Even when conformational isomers are produced, such isomers and mixtures thereof are encompassed in compound (1) and compound (2) of the present invention.

In addition, the above-mentioned compound (1) and compound (2) may form a stable chelating compound with a metal salt, such as Ag, Mn, Zn and the like. As explained in the following, compound (2) is a precursor (synthesis intermediate) of compound (1).

Now, the production methods of compound (1) and compound (2) are explained. Compound (1) and compound (2), or a salt thereof can be produced, for example, as follows. Compound (1) and compound (2), or a salt thereof and starting material compounds thereof can be produced using a method known *per se*, for example, a method shown by the following scheme and the like. In the following description, the "room temperature" generally means 10°C to 30°C and, unless otherwise specified, each symbol in the chemical structural formulas described in the schemes is as defined above. The compounds in the formulas include salts unless otherwise specified, and examples of such salt include those similar to the salts of compound (1) and compound (2), and the like.

The compound obtained in each step can be used for the next reaction in the form of a reaction mixture or a crude product. It can also be isolated from a reaction mixture according to a conventional method, and can be easily purified by a separation means such as recrystallization, distillation, chromatography and the like.

The compound (2) of the present invention can be produced, for example, using the following production method 1 and production method 3, and compound (1) of the present invention can be produced, for example, using the following production method 2 and production method 4. The starting compound of each method may be a commercially available product, or can also be produced from the corresponding compound by a method known *per se* to those of ordinary skill in the art.

### Production method 1

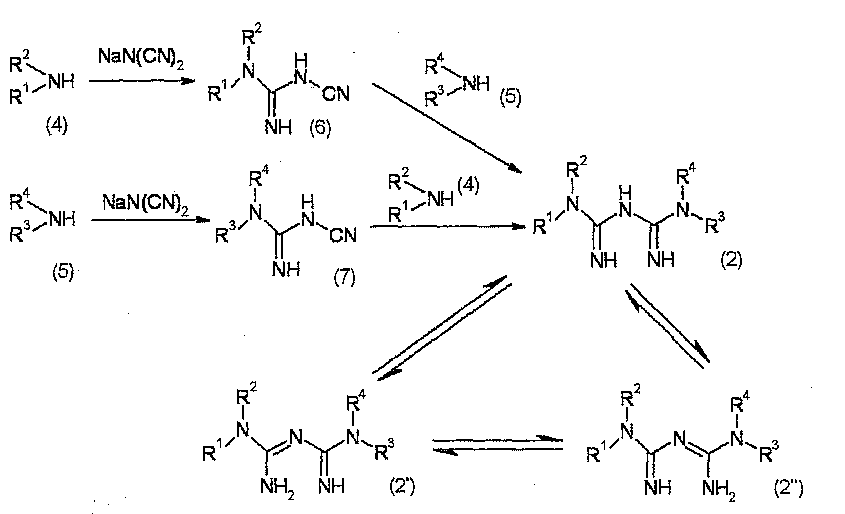

The production method (production method 1) of compound (2), compound (2') or compound (2"), which is a precursor of compound (1), is shown. According to this method, compound (4) or (5) is firstly reacted with an acid for conversion to an acid addition salt, which is then reacted with sodium dicyanamide in a solvent to give a cyanoguanidine derivative (compound (6) or (7)). The compound (2') and compound (2") are tautomers of compound (2).

The conversion to an acid addition salt can be performed by a method known *per se.* Examples of the acid to be used include hydrochloric acid, sulfuric acid, p-toluenesulfonic acid, benzenesulfonic acid, methanesulfonic acid and the like. The amount of the acid to be used is about 1 to 2 mol, preferably about 1 to 1.5 mol, per 1 mol of compound (4) or compound (5), and the reaction temperature is generally about 0°C to 100°C, preferably about 0°C to 30°C.

The solvent may be any as long as it does not influence the reaction and, for example, methanol, ethanol, propanol, isopropanol, butanol, benzene, toluene, xylene, ethyl acetate, tetrahydrofuran, acetonitrile, N,N-dimethylformamide and the like can be mentioned.

The amount of sodium dicyanamide to be used is about 1 to 2 mol, preferably about 1 to 1.3 mol, per 1 mol of compound (4) or (5), and the reaction temperature is generally about 60°C to 150°C, preferably about 75°C to 120°C.

Without conversion of compounds (4) and (5) to acid addition salts, by reaction with sodium dicyanamide in the presence of an equivalent of an acid (for example, acid such as hydrochloric acid, sulfuric acid, p-toluenesulfonic acid, benzenesulfonic acid, methanesulfonic acid and the like), compounds (6) and (7) can be produced in the same manner.

Then, a biguanide derivative (compound (2)) can be produced by reacting compound (6) or (7) with compound (5) or compound (4), respectively, in a solvent in the presence of an acid.

Examples of the acid include acids such as hydrochloric acid, sulfuric acid, p-toluenesulfonic acid, benzenesulfonic acid, methanesulfonic acid and the like, and those of ordinary skill in the art can select the acid as appropriate. The amount of the acid to be used is about 1 to 2 mol, preferably about 1 to 1.3 mol, per 1 mol of compound (6) or compound (7). As the solvent, any can be used as long as it does not influence the reaction and, for example, methanol, ethanol, propanol, isopropanol, butanol, benzene, toluene, xylene, mesitylene, ethyl acetate, tetrahydrofuran, acetonitrile, N,N-dimethylformamide and the like can be mentioned.

The amount of compound (5) or (4) to be used is about 1 to 2 mol, preferably about 1 to 1.3 mol, each per 1 mol of compound (6) or (7). The reaction temperature is generally about 60°C to 170°C, preferably about 110°C to 150°C.

The resulting compound (2) can be obtained in the form a salt with an acid used. Where necessary, neutralization is performed using sodium hydroxide, potassium hydroxide and the like to give a free base. Compound (2) can be used for the next reaction in the form of an acid addition salt or a free base, without purification.

### Production method 2

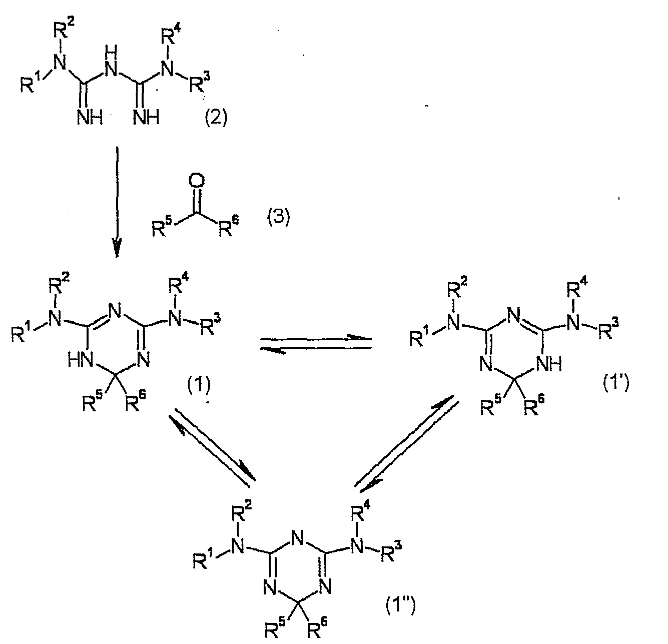

Then, a method of producing compound (1), compound (1') or compound (1") by reacting compound (2) with a compound represented by the formula (3) in the presence of acid is shown. The compound (1') and compound (1") are tautomers of compound (1).

As compound (3), ketone, aldehyde, as well as equivalents thereof, for example, acetals and the like, can be used.

This reaction is performed in the presence of acid using compound (3) as a solvent, or a mixture of compound (3) and other solvent as a solvent.

When compound (3) is used as a solvent in this reaction, the amount of compound (3) to be used is about 1 to 12 mol, preferably about 1 to 2 mol, per 1 mol of compound (2), and the reaction temperature is generally ambient temperature to about 150°C, preferably about 60°C to 80°C.

When a mixture of compound (3) and other solvent is used as a solvent in this reaction, the amount of compound (3) to be used is about 1 to 12 mol, preferably about 1 to 2 mol, per 1 mol of compound (2), and the reaction temperature is generally ambient temperature to about 150°C, preferably about 60°C to 80°C. Examples of other solvent include solvents such as methanol, ethanol, propanol, isopropanol, butanol, ethyl acetate, tetrahydrofuran, acetonitrile, N,N-dimethylformamide and the like, and a mixed solvent thereof.

Examples of the acid include hydrochloric acid, sulfuric acid, camphorsulfonic acid, p-toluenesulfonic acid, benzenesulfonic acid, methanesulfonic acid and the like. The amount of the acid to be used is about 0.1 to 1.5 mol, preferably about 0.15 to 0.6 mol, per 1 mol of compound (2), and the reaction temperature is generally room temperature to about 150°C, preferably about 60°C to 80°C.

The obtained crude compound (1) is heated in water or an aqueous solvent (for example, aqueous solvent such as methanol, ethanol, propanol, isopropanol, tetrahydrofuran, acetonitrile and the like) containing a base (for example, sodium hydroxide, potassium hydroxide etc.) to give a free base of compound (1). The amount of the base to be used and water content of the aqueous solvent can be appropriately determined by those of ordinary skill in the art. The reaction temperature is generally about 50°C to 100°C, preferably about 80°C to 100°C.

### Production method 3

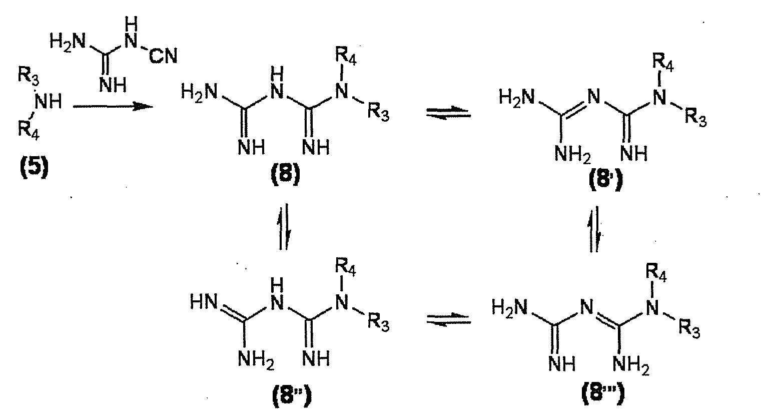

A method of producing precursor for compound (1), namely compound (8), compound (8'), compound (8") or compound (8'"), wherein R¹ and R² are each a hydrogen atom, is shown. According to this method, compound (5) is first reacted with an acid for conversion to an acid addition salt, which is then reacted with dicyandiamide in a solvent to give a biguanide derivative compound (8). The compounds (8'), (8") and compound (8"') are tautomers of compound (8).

The compound (5) is converted to an acid addition salt (e.g., salts with hydrochloric acid, sulfuric acid, p-toluenesulfonic acid, benzenesulfonic acid, methanesulfonic acid and the like), which is then reacted with dicyandiamide without solvent, or in a solvent (e.g., methanol, ethanol, propanol, isopropanol, butanol, benzene, toluene, xylene, ethyl acetate, tetrahydrofuran, acetonitrile, N,N-dimethylformamide and the like) to give a biguanide derivative compound (8). Without converting compound (5) to acid addition salt, compound (8) can also be produced by reaction with dicyandiamide in the presence of an equivalent of an acid (e.g., hydrochloric acid, sulfuric acid, p-toluenesulfonic acid, benzenesulfonic acid, methanesulfonic acid and the like) in the same manner.

The amount of dicyandiamide to be used is about 1 to 2 mol, preferably about 1 to 1.3 mol, per 1 mol of compound (5) and the reaction temperature is generally about 60°C to 200°C. Without a solvent, it is preferably about 150 to 200°C and, when a solvent is used, it is preferably about 80°C to 100°C.

While the resulting compound (8) is obtained in the form of a salt with an acid, where necessary, it can also be recovered in the form of a free base by neutralization with sodium hydroxide, potassium hydroxide and the like. Without purification, compound (8) can also be used in the form of an acid addition salt or free base for the next reaction.

### Production method 4

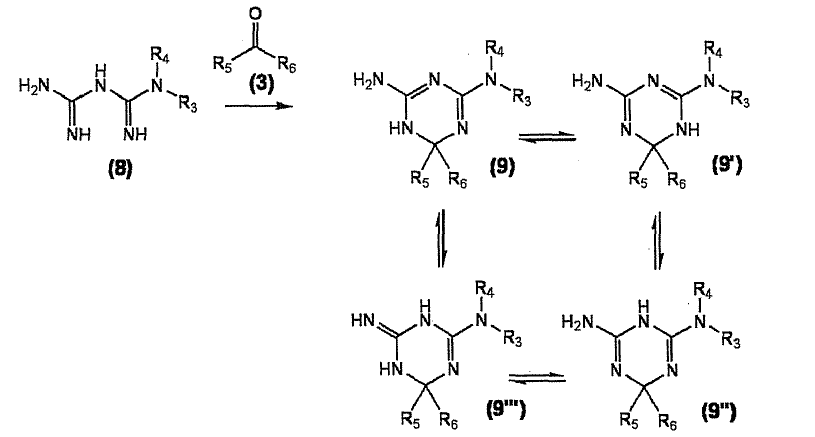

A method of producing compound (9), (9'), (9") or (9"'), wherein R¹ and R² are each a hydrogen atom is shown. The reaction of compound (8) with compound (3) can be performed in the same manner as in the aforementioned reaction of compound (2) and compound (3). The compounds (9'), (9") and (9"') are tautomers of compound (9).

A free base of compound (1), (2), (8) or (9) obtained as shown above can be led to an appropriate organic or inorganic acid addition salt by using the aforementioned acid or acid salt (for example, sodium chloride, sodium bromide, sodium acetate, potassium acetate, sodium nitrate, potassium nitrate etc.) or lactone (gluconolactone etc.) in water, a solvent (for example, ethanol, methanol, acetonitrile, acetone, methyl ethyl ketone etc.) or an aqueous solvent. These acid addition salts can also be purified by an isolation and purification means known *per se* such as recrystallization, chromatography and the like.

When compounds (1), (2), (8) and (9) contain an optical isomer, a stereoisomer, a positional isomer or a rotational isomer, these are also encompassed in the compounds (1), (2), (8) and (9), and can be obtained as a single product according to a synthesis method and separation method known *per se* (e.g., concentration, solvent extraction, column chromatography, recrystallization etc.). For example, when compounds (1), (2), (8) and (9) have an optical isomer, an optical isomer resolved from these compounds is also encompassed in compounds (1), (2), (8) and (9).

The optical isomer can be produced by a method known *per se*. To be specific, an optically active synthetic intermediate is used, or the final racemate product is subjected to optical resolution according to a conventional method to give an optical isomer.

The method of optical resolution may be a method known *per se*, such as a fractional recrystallization method, a chiral column method, a diastereomer method and the like.

The compound (1) and compound (2) of the present invention has a superior antibacterial action, particularly an extremely strong antibacterial action even by a treatment for a short time. Therefore, the present invention provides a bactericidal/disinfectant agent containing compound (1) or compound (2) as an active ingredient. The bactericidal/disinfectant agent of the present invention shows a strong growth inhibitory effect and a bactericidal effect particularly against wide spectrum of gram positive bacteria and gram negative bacteria even by a treatment for a short time.

The agent of the present invention containing compound (1) or compound (2) as an active ingredient is extremely useful as an external bactericidal/disinfectant agent. For example, the agent can be used not only for sterilization or disinfection of a wound site, a burn site or a bedsore site, or for sterilization or disinfection of an operation site before and after operation, but also for sterilization or disinfection of hands and arms of medical employees, or sterilization or disinfection of medical equipments and medical environment (construction and facilities thereof). In addition, the agent of the present invention containing compound (1) or compound (2) as an active ingredient is useful for human as well as animals (e.g., mammals such as dog, cat, sheep, swine, horse, bovine and the like), birds (e.g., chicken, dabbling duck, duck, quail, turkey, and the like), and fish (e.g., sea bream, young yellowtail, eel, and the like).

The agent of the present invention containing compound (1) or compound (2) as an active ingredient can also be used, for example, as an antiseptic/preservative agent for cosmetics (cream, emulsion, toner etc.).

Moreover, the compound of the present invention is extremely superior in solubility in water than conventional structurally-similar compounds, and superior in storage stability in a high concentration solution state. Therefore, the compound is dissolved in a smaller amount of water, can be transported in a high concentration solution state, can be appropriately diluted when in use as a bactericidal/disinfectant agent, and enables use at a concentration necessary for the situation. The drastically improved solubility in water of the compound of the present invention as compared to structurally-similar known compounds is shown in the below-mentioned Experimental Example 2.

For application of a bactericidal/disinfectant agent, an antiseptic/preservative agent for cosmetics and the like to the skin, the sensitivity (allergenicity) is desirably as small as possible. The compound of the present invention shows extremely low skin sensitivity, and is particularly suitable for use as a bactericidal/disinfectant agent, an antiseptic/preservative agent for cosmetics and the like. The extremely low skin sensitivity of the compound of the present invention as compared to structurally-similar known compounds is shown in the below-mentioned Experimental Example 3.

As a medicine of the present invention, the compound (1) or compound (2) or a pharmacologically acceptable salt thereof may be used as it is, but generally a form of a medical preparation containing the aforementioned active ingredients and 1 or 2 or more pharmaceutical additives is preferable. Examples of such pharmaceutical preparation include powders, suppositories, paste agents, ointments, creams, gels, gel-like creams, lotions, emulsions, suspensions, poultices, plasters, liniments, aerosols, syrups, oral cavity agents, eye drops, nasal drops and the like. Inter alia, the medicine of the present invention preferably has a dosage form of external liquids.

The aforementioned pharmaceutical preparation can be prepared by the method which is known *per se* or conventional in the field of pharmacy.

When the medicine of the present invention has a dosage form of ointments, the medicine of the present invention can be prepared by incorporating the compound (1) or the compound (2) oral pharmacologically acceptable salt thereof and, optionally, preparation additives such as emulsifying agents such as anionic or nonionic surfactants, and preservatives such as paraoxybenzoic acid esters into bases such as lipophilic bases such as vaseline, liquid paraffin, silicone and vegetable oil; emulsion bases such as hydrophilic vaseline and purified lanoline; or water-soluble bases such as macrogol.

When the medicine of the present invention has a dosage form of gels, the medicine of the present invention can be prepared by incorporating the compound (1) or the compound (2) or a pharmacologically acceptable salt thereof and, optionally, preparation additives such as lower alcohols, neutralizing agents, surfactants and absorption promoters into a base obtained by adding a gelling agent (e.g. carboxyvinyl polymer, hydroxyethylcellulose, hydroxypropylcellulose, methylcellulose, ethylcellulose, carboxymethylcellulose and alginic acid propylene glycol ester, etc.) to water.

When the medicine of the present invention has a dosage form of creams, the medicine of the present invention can be prepared by incorporating the compound (1) or compound (2) or a pharmacologically acceptable salt thereof and, optionally, preparation additives such as emulsifying agents, antiseptics, absorption promoters and rash preventing agents into a base containing fatty acid esters (e.g. myristic acid ester, palmitic acid ester, diethyl sebacate, hexyl laurate, cetyl isooctate, etc.), lower alcohols (e.g. ethanol, isopropanol, etc.), hydrocarbons (e.g. liquid paraffin, squalane, etc.), polyhydric alcohols (e.g. propylene glycol, 1,3-butylene glycol, etc.) or higher alcohols (e.g. 2-hexyldecanol, cetanol, 2-octyldodecanol, etc.).

In addition, in order to obtain gel-like creams having a nature between creams and gels, a gelling agent and a neutralizing agent may be added to the aforementioned creams.

When the medicine of the present inventions has a dosage form of external liquids, the medicine of the present invention can be prepared by incorporating the compound (1) or compound (2) or a pharmacologically acceptable salt thereof and, optionally, preparation additives such as buffers, stabilizers, antiseptics, pH adjusting agents, solvents, solubilizers, flavors, gels, corrigents and refreshing agents into a solvent. Examples of such solvent include, for example, glycerin, propylene glycol, ethanol, isopropanol, butylene glycol, water, sorbitol, mannitol, xylitol, glucose, ε-aminocaproic acid, glycine, glutamic acid salt, sodium hyaluronate, polyethylene glycols, carboxyvinyl polymers, higher alcohols such as cetanol and stearyl alcohol, fatty acid esters such as medium-chain fatty acid esters and isopropyl mysristate, higher fatty acid such as stearic acid, squalane, liquid paraffin, white vaseline and purified lanolin.

Herein, examples of external liquids include liquid preparations which are subjected to external use such as washing, injection, wet compression, inhalation, spraying, enema administration, coating, drug bathing, clean wiping, disinfection, eye dropping, eye washing, ear dropping, nasal dropping and the like.

Aerosols can be prepared by using external liquids of the present invention together with a normal propellant. Examples of the propellant include dimethyl ether, liquefied petroleum gas, N₂ gas, nitrous oxide gas, CO₂ gas, and alternative chlorofluorocarbon gas. The compressed air may be used without using a propellant. Alternatively, a mixture of them may be used.

For the external bactericidal/disinfectant agent of the present invention, it is preferable that a dose is adjusted so that an active ingredient is 0.01 to 10% by weight.

### Examples

The present invention is explained in more detail in the following by referring to Reference Examples and Examples, which are not to be construed as limitative.

### Reference Example 1 N¹-octyl-N¹-methyl-cyanoguanidine

Under ice-cooling, to a suspension of N-methyloctylamine (35.0 g, 0.24 mol) and sodium dicyanamide (26.1 g, 0.29 mol) in xylene (320 ml) and DMF (80 ml) was added concentrated sulfuric acid (12.1 g), and the mixture was stirred for 7 hr with heating at 80-85°C. Under ice-cooling, water was added, and the precipitated crystals were collected by filtration, washed with water, and dried under reduced pressure to give colorless crystals (21.6 g) with melting point of 101 to 104°C. Further, the xylene layer was washed with water, dried over anhydrous magnesium sulfate, concentrated and cooled. The precipitated crystals were collected by filtration to give the title compound (17.1 g) as colorless crystals.
¹H-NMR(DMSO-d₆) δ: 0.86 (3H, t, J=6Hz,CH₃), 1.1-1.5 (12H, m, (CH₂)₆), 2.85 (3H, s, CH₃NCH₂), 3.24 (2H, t, J=7Hz, CH₃NCH₂), 6.91(2H, s, NH×2).

### Reference Example 2 N¹-benzyl-N¹-methyl-cyanoguanidine

To N-methylbenzylamine hydrochloride (45.0 g, 0.29 mol) were added sodium dicyanamide (26.0 g, 0.29 mol), 1-butanol (281 ml) and water (22.5 ml) and the mixture was heated under reflux for 7 hr. The precipitated sodium chloride was filtered off, and the solvent of the filtrate was evaporated under reduced pressure. The residue was washed with water and dried under reduced pressure to give the title compound (52.0 g) as colorless crystals.
melting point: 102 - 105°C
¹H-NMR (DMSO-d₆) δ: 2.85 (3H, s, CH₃N), 4.55 (2H, s, ArCH₂), 7.1-7.4 (7H, m, ArH and NH×2).

### Reference Example 3 4-octylamino-2-cyclohexylmethylamino-1,6-dihydro-6,6-dimethyl-1,3,5-triazine acetate

A crude compound obtained according to the synthesis methods described in Reference Example 4 and Reference Example 5 of patent document 8 and Example 1 of the present invention and using cyclohexylmethylamine as a starting material was purified by silica gel column chromatography (eluent, mixed solution of chloroform:methanol:acetic acid = 8:1:1) to give the title compound as colorless crystals.
melting point: 70-73°C
¹H-NMR (CDCl₃) δ: 0.88(3H, t, J=7Hz, CH₃), 0.8-1.8 (23H, m, cyclohexyl, (CH₂)₆), 1.36 (6H, s, (CH₃)₂C), 1.97 (3H, s, CH₃COO⁻), 3.16, 3.27 (each 2H, m, NHCH₂'2), 8.15 (2H, m, NH'2), 9.13 (2H, br s, NH, NH⁺).

### Reference Example 4 1,6-dihydro-6,6-dimethyl-4-octylamino-2-phenylamino-1,3,5-triazine acetate

A crude compound obtained according to the synthesis methods described in Reference Example 1 and Reference Example 2 of patent document 8 and Example 1 of the present invention and using aniline as a starting material was purified by silica gel column chromatography (eluent, mixed solution of chloroform:methanol:acetic acid = 9:1:1) to give the title compound as colorless crystals.
melting point: 122-124°C
¹H-NMR (CDCl₃) δ: 0.87 (3H, t, J=7Hz, CH₃), 1.2-1.4 (10H, m), 1.43 (6H, s, (CH₃)₂C), 1.53 (2H, m, NHCH₂CH₂), 2.03 (3H, s, CH₃COO⁻), 3.29(2H, br dt-like, NHCH₂CH₂), 7.0-7.6 (5H, m, ArH).

### Reference Example 5 4-octylamino-1,6-dihydro-6,6-dimethyl-2-(4-methylbenzylamino-1,3,5-triazine gluconate

The title compound was obtained according to the synthesis method described in Example 77 of patent document 8.
melting point: 122-124°C
¹H-NMR (DMSO-d₆-D₂₀) δ: 0.85 (3H, t, J=7Hz, CH₃), 1.1-1.5 (12H, m), 1.37 (6H, s, (CH₃)₂C), 2.27 (3H, s, ArCH₃), 3.0-3.9 (6H, m, gluconic acid), 3.20 (2H, br. t-like, NHCH₂), 4.41 (2H, s, ArCH₂), 7.12, 7.17 (each 2H, d, J = 8Hz, ArH).

### Example 1

### N⁴-octyl-N⁴-methyl-1,6-dihydro-6,6-dimethyl-N²-(4-chlorobenzyl)-1,3,5-triazine-2,4-diamine acetate

To N⁵-octyl-N⁵-methyl-N¹-(4-chlorobenzyl)-biguanide dihydrochloride (Example 27, 11.0 g, 25.9 mmol) were added ethanol (145 ml), acetone (36 ml) and concentrated hydrochloric acid (0.7 ml) and the mixture was refluxed for 48 hr. The solvent was evaporated under reduced pressure, and the residue was dissolved in ethanol (110 ml). Water (70 ml) and 5N aqueous sodium hydroxide solution (12.5 ml) were added, and the mixture was refluxed for 2 hr, concentrated under reduced pressure, and extracted with ethyl acetate. The extract was washed with 5% aqueous sodium acetate solution and water, and the solvent was evaporated under reduced pressure. The residue was recrystallized from methyl ethyl ketone to give the title compound (5.5 g) as colorless crystals.
melting point: 94-96°C
¹H-NMR (DMSO-d₆) δ: 0.84 (3H, t, J=6Hz, CH₃), 1.0-1.5 (12H, m, (CH₂)₆), 1.36 (6H, s, CH₃×2), 1.66 (3H, s, CH₃COO⁻), 2.92 (3H, s, NCH₃), 3.37 (2H, t, J=7Hz, CH₃NCH₂), 4.38 (2H, s, ArCH₂NH), 7.25 (2H, d, J=8Hz, ArH), 7.36 (2H, d, J=8Hz, ArH).

### Example 2

### N⁴-octyl-N⁴-methyl-1,6-dihydro-6,6-dimethyl-N²-(3,4-dichlorobenzyl)-1,3,5-triazine-2,4-diamine acetate

To N⁵-octyl-N⁵-methyl-N¹-(3,4-dichlorobenzyl)-biguanide hydrochloride (Example 28, 10.0 g, 23.7 mmol) were added ethanol (145 ml), acetone (36 ml) and concentrated sulfuric acid (1.51 g) and the mixture was refluxed for 48 hr. The solvent was evaporated under reduced pressure. The residue was dissolved in ethanol (110 ml), water (70 ml) and 5N aqueous sodium hydroxide solution (11.4 ml) were added, and the mixture was refluxed for 2 hr, concentrated under reduced pressure, and extracted with ethyl acetate. The extract was washed with 5% aqueous sodium acetate solution and water, and the solvent was evaporated under reduced pressure. The residue was recrystallized from methyl ethyl ketone to give the title compound (1.5 g) as colorless crystals.
melting point: 73-76°C
¹H-NMR (DMSO-d₆) δ: 0.84 (3H, t, J=6Hz, CH₃), 1.0-1.4 (12H, m, (CH₂)₆), 1.38 (6H, s, CH₃×2), 1.67 (3H, s, CH₃COO⁻), 2.93 (3H, s, NCH₃), 3.37 (2H, t, J=7Hz, CH₃NCH₂), 4.40 (2H, s, ArCH₂NH), 7.23 (H, d,d, J=2,8Hz, ArH), 7.47 (H, d, J=2Hz, ArH), 7.58 (H, d, J=8Hz, ArH).

### Example 3

### 1,6-dihydro-6,6-dimethyl-N⁴-decyl-N²-benzyl-N²-methyl-1,3,5-triazine-2,4-diamine gluconate

To N⁵-decyl-N¹-benzyl-N¹-methyl-biguanide dihydrochloride (Example 29, 12.0 g, 28.7 mmol) were added ethanol (160 ml), acetone (40 ml) and concentrated hydrochloric acid (0.7 ml) and the mixture was refluxed for 48 hr. The solvent was evaporated under reduced pressure and the residue was dissolved in ethanol (120 ml). Water (75 ml) and 5N aqueous sodium hydroxide solution (13.6 ml) were added, and the mixture was refluxed for 2 hr, concentrated under reduced pressure, and extracted with ethyl acetate. The extract was washed with 5% aqueous sodium acetate solution and water, and the solvent was evaporated under reduced pressure. The residue (acetate) was purified by silica gel column chromatography (eluent, chloroform:methanol:acetic acid = 8.5:1:1). To the purified fraction (9.1 g) were added toluene (50 ml) and 2.5N aqueous sodium hydroxide solution (14 ml), and the mixture was stirred, and extracted with toluene. The extract was washed with water, and the solvent was evaporated under reduced pressure to give a syrupy solid (base) (6.7 g). Then, the syrupy solid (base) (6.7 g)(17.4 mmol) was dissolved in methyl ethyl ketone (40 ml), 50% aqueous gluconic acid solution (7.1 g) was added, and the mixture was stirred at 60°C for 3 hr. The solvent was evaporated under reduced pressure, and a mixed solution of toluene and ethanol was added. The solvent was evaporated under reduced pressure to remove water. The residue was sufficiently dried under reduced pressure to give the title compound (10.5 g) as a pale-yellow syrupy solid.
¹H-NMR (DMSO-d₆-D₂O) δ: 0.85 (3H, t, J=6Hz, CH₃), 1.1-1.5 (16H, m, (CH₂)₈), 1.43 (6H, s, CH₃×2), 2.97 (3H, s, ArCH₂NCH₃), 3.1-3.9 (6H, m, gluconic acid), 3.20 (2H, m, HNCH₂), 4.75 (2H, s, ArCH₂NCH₃), 7.2-7.4 (5H, m, ArH).

### Example 4

### 1,6-dihydro-6,6-dimethyl-N⁴-nonyl-N²-benzyl-N²-methyl-1,3,5-triazine-2,4-diamine acetate

To N⁵-nonyl-N¹-methyl-N¹-benzyl-biguanide dihydrochloride (Example 30, 11.5 g, 28.4 mmol) were added ethanol (160 ml), acetone (40 ml) and (±)-10-camphorsulfonic acid (0.66 g) and the mixture was refluxed for 24 hr. The solvent was evaporated under reduced pressure and the residue was dissolved in ethanol (120 ml). Water (75 ml) and 5N aqueous sodium hydroxide solution (13.5 ml) were added, and the mixture was refluxed for 2 hr, concentrated under reduced pressure, and extracted with ethyl acetate. The extract was washed with 5% aqueous sodium acetate solution and water, and the solvent was evaporated under reduced pressure. The residue was purified by silica gel column chromatography (eluent, mixed solution of chloroform:methanol:acetic acid = 8:1:1) to give the title compound (7.3 g) as a pale-yellow syrupy solid.
¹H-NMR (CDCl₃) δ: 0.86 (3H, t, J=6Hz, CH₃), 1.1-1.6 (14H, m, (CH₂)₇), 1.48 (6H, s, GH₃×2), 1.96 (3H, s, CH₃COO⁻), 3.03 (3H, s, NCH₃), 3.25 (2H, br.dt-like, HNCH₂), 4.75 (2H, s, ArCH₂NH), 7.2-7.4 (6H, m, ArH and NH), 7.85, 9.42 (each 1H, br.m, NH, NH⁺).

### Example 5

### N⁴-octyl-1,6-dihydro-6,6-dimethyl-N²-benzyl-N²-methyl-1,3,5-triazine-2,4-diamine acetate

To N⁵-octyl-N¹-benzyl-N¹-methyl-biguanide dihydrochloride (Example 31, 15.0 g, 38.4 mmol) were added methanol (240 ml), acetone (60 ml) and concentrated hydrochloric acid (1.0 g) and the mixture was refluxed for 24 hr. The solvent was evaporated under reduced pressure and the residue was dissolved in ethanol (160 ml). Water (100 ml) and 5N aqueous sodium hydroxide solution (18.2 ml) were added, and the mixture was refluxed for 2 hr, concentrated under reduced pressure, and extracted with ethyl acetate. The extract was washed with 5% aqueous sodium acetate solution and water. The solvent was evaporated under reduced pressure and the residue was purified by silica gel column chromatography (eluent, mixed solution of chloroform:methanol:acetic acid = 8:1.5:1) to give the title compound (5.9 g) as a pale-yellow syrupy solid.
¹H-NMR (CDCl₃) δ: 0.85 (3H, t, J=6Hz, CH₃), 1.1-1.6 (12H, m, (CH₂)₆), 1.51 (6H, s, CH₃×2), 1.93 (3H, s, CH₃COO⁻), 3.06 (3H, s, ArCH₂NCH₃), 3.25 (2H, t, J=7Hz, HNCH₂), 4.75 (2H, s, ArCH₂NCH₃), 7.2-7.4 (8H, m, ArH and NH×2, NH⁺).

### Example 6

### N⁴-octyl-N⁴-methyl-1,6-dihydro-6,6-dimethyl-N²-benzyl-N²-methyl-1,3,5-triazine-2,4-diamine acetate

To N⁵-octyl-N⁵-methyl-N¹-benzyl-N¹-methyl-biguanide dihydrochloride (Example 32, 13.0 g, 32.1 mmol) were added ethanol (180 ml), acetone (45 ml) and concentrated hydrochloric acid (0.8 ml) and the mixture was refluxed for 48 hr. The solvent was evaporated under reduced pressure and the residue was dissolved in ethanol (120 ml). Water (75 ml) and 5N aqueous sodium hydroxide solution (15.3 ml) were added, and the mixture was refluxed for 2 hr, concentrated under reduced pressure, and extracted with toluene. The extract was washed with water, and the solvent was evaporated under reduced pressure. To the residue were added 4N mixed solution of hydrochloric acid and ethyl acetate (16 ml), and the solvent was evaporated under reduced pressure. The residue was purified by silica gel column chromatography (mixed solution of chloroform:methanol:acetic acid = 8:1.5:1) to give the title compound (4.6 g) as a pale-yellow syrupy solid. ¹H-NMR (CDCl₃) δ: 0.85 (3H, t, J=6Hz, CH₃), 1.1-1.6 (12H, m, (CH₂)₆), 1.84 (6H, s, CH₃×2), 3.16, 3.20 (each 3H, s, NCH₃×2), 3.41 (2H, m, CH₃NCH₂), 4.74 (2H, s, ArCH₂NCH₃), 7.1-7.3 (6H, m, ArH and NH), 8.32, 8.51 (each H, m, NH and NH⁺).

### Example 7

### N⁴-octyl-N⁴-methyl-1,6-dihydro-6,6-dimethyl-N²-(4-methylbenzyl)-1,3,5-triazine-2,4-diamine acetate

To N⁵-octyl-N⁵-methyl-N¹-(4-methylbenzyl)-biguanide hydrochloride (Example 33, 10.0 g, 27.2 mmol) were added ethanol (145 ml), acetone (36 ml) and concentrated sulfuric acid (1.8 ml) and the mixture was refluxed for 24 hr, and concentrated under reduced pressure. Ethanol (64 ml), water (70 ml) and 5N aqueous sodium hydroxide solution (13.5 ml) were added, and the mixture was refluxed for 1 hr, concentrated under reduced pressure, and extracted with ethyl acetate. The extract was washed with 5% aqueous sodium acetate solution and water, and the solvent was evaporated under reduced pressure. The residue was purified by silica gel column chromatography (eluent, mixed solution of chloroform:methanol:acetic acid = 8:0.9:0.9) to give the title compound (9.9 g) as a pale-yellow syrupy solid.
¹H-NMR (CDCl₃) δ: 0.87 (3H, t, J=6Hz, CH₃), 1.0-1.6 (12H, m, (CH₂)₆), 1.49 (6H, s, CH₃×2), 1.92 (3H, s, CH₃COO⁻), 2.30 (3H, s, ArCH₃), 2.99 (3H, s, NCH₃), 3.40 (2H, t, J=7Hz, CH₃NCH₂), 4.42 (2H, s, ArCH₂NH), 7.06 (2H, d, J=8Hz, ArH), 7.15 (2H, d, J=8Hz, ArH).

### Example 8

### 1,6-dihydro-N-dodecyl-4-(morpholin-4-yl)-1,3,5-triazine-2-amine

To N-dodecyl-N'-(imino-morpholin-4-yl-methyl) guanidine hydrochloride (Example 34, 1.0 g, 2.6 mmol) were added ethanol (15 ml), acetone (4 ml) and concentrated sulfuric acid (170 mg, 1.7 mmol) and the mixture was refluxed for 20 hr. The solvent was evaporated under reduced pressure. Ethanol (11 ml), water (7 ml) and 5N aqueous sodium hydroxide solution (11 ml) were added, and the mixture was refluxed for 2 hr, and concentrated under reduced pressure. The precipitated crystals were collected by filtration, washed with water and recrystallized from methyl ethyl ketone to give the title compound (380 mg) as colorless crystals.
melting point: 117-118°C
¹H-NMR (CDCl₃) δ: 0.88 (3H, t, J=6Hz, CH₃), 1.2-1.6 (20H, m, (CH₂)₁₀), 1.34 (6H, s, CH₃×2), 3.20 (2H, t, J=7Hz, HNCH₂), 3.46 (4H, t, J=5Hz, NCH₂CH₂O×2), 3.70 (4H, t, J=5Hz, NCH₂CH₂O×2).

### Example 9

### N-dodecyl-6,6-dimethyl-4-(morpholin-4-yl)-1,6-dihydro-1,3,5-triazine-2-amine acetate

1,6-Dihydro-N-dodecyl-4-(morpholin-4-yl)-1,3,5-triazine-2-amine (Example 8, 5.0 g, 13.2 mmol) was dissolved in a mixed solution of ethyl acetate (120 ml) and water (30 ml), and the mixture was sufficiently stirred. Ethyl acetate was evaporated under reduced pressure, and water was removed by 5 repeats of azeotropic distillation with toluene. The precipitated crystals were recrystallized from methyl ethyl ketone to give the title compound (5.6 g) as colorless crystals.
melting point: 120-122°C
¹H-NMR (CDCl₃) δ: 0.88 (3H, t, J=6Hz, CH₃), 1.2-1.6 (20H, m, (CH₂)₁₀), 1.46 (6H, s, CH₃×2), 1.96 (3H, s, CH₃COO⁻), 3.2-3.3 (2H, m, HNCH₂), 3.72 (8H, s, NCH₂CH₂O×2), 9.0-9.1 (2H, br, NH), 9.9-10.1 (1H, br, NH⁺).

### Example 10

### N-dodecyl-6,6-dimethyl-4-(4-methylpiperazin-1-yl)-1,6-dihydro-1,3,5-triazine-2-amine acetate

To N-dodecyl-N'-(imino-4-methylpiperazin-1-yl-methyl)guanidine hydrochloride (Example 35, 1.5 g, 3.9 mmol) were added ethanol (23 ml), acetone (6 ml) and concentrated sulfuric acid (435 mg, 4.4 mmol) and the mixture was refluxed for 24 hr. The solvent was evaporated under reduced pressure. Ethanol (15 ml), water (10 ml) and 5N aqueous sodium hydroxide solution (17 ml) were added, and the mixture was refluxed for 2 hr, concentrated under reduced pressure, and extracted with ethyl acetate. The extract was washed withed 5% aqueous sodium acetate solution and water and the solvent was concentrated under reduced pressure. The residue was purified by silica gel column chromatography (eluent, mixed solution of chloroform:methanol = 10:1) to give the title compound (300 mg) as a pale-yellow syrupy solid.
¹H-NMR (CDCl₃) δ: 0.88 (3H, t, J=6Hz, CH₃), 1.2-1.6 (20H, m, (CH₂)₁₀), 1.46 (6H, s, CH₃×2), 1.95 (3H, s, CH3COO⁻), 2.31 (3H, s, NCH₃), 2.4-2.5 (4H, br, CH₃NCH₂CH₂N×2), 3.25 (2H, t, J=7Hz, HNCH₂), 3.6-3.7 (4H, br, CH₃NCH₂CH₂N×2).

### Example 11

### N-decyl-6,6-dimethyl-4-(piperidin-1-yl)-1,6-dihydro-1,3,5-triazine-2-amine acetate

To N-decyl-N'-(imino-piperidin-1-yl-methyl)guanidine hydrochloride (Example 36, 1.0 g, 2.9 mmol) were added ethanol (15 ml), acetone (4 ml) and concentrated sulfuric acid (184 mg, 1.9 mmol) and the mixture was refluxed for 24 hr. The solvent was evaporated under reduced pressure. Ethanol (11 ml), water (7 ml) and 5N aqueous sodium hydroxide solution (11 ml) were added,'and the mixture was refluxed for 2 hr, concentrated under reduced pressure, and extracted with ethyl acetate. The extract was washed with 5% aqueous sodium acetate solution and water. The solvent was concentrated under reduced pressure, and the residue was purified by silica gel column chromatography (eluent, mixed solution of chloroform:methanol = 10:1) to give the title compound (315 mg) as a pale-yellow solid.
¹H-NMR (CDCl₃) δ: 0.88 (3H, t, J=6Hz, CH₃), 1.2-1.7 (22H, m, N(CH₂)₂(CH₂)₃ and (CH₂)₈), 1.47 (6H, s, CH₃×2), 1.96 (3H, s, CH₃COO⁻), 3.2-3.3 (2H, m, HNCH₂), 3.6-3.7 (4H, m, N(CH₂)₂(CH₂)₃), 8.05 (1H, br, NH), 9.30 (1H, br, NH), 10.35 (1H, br, NH⁺).

### Example 12

### N-decyl-4-(1,3-dihydro-2H-isoindal-2-yl)-6,6-dimethyl-1,6-dihydro-1,3,5-triazine-2-amine

To N-decyl-N'-(imino-1,3-dihydro-2H-isoindol-2-yl-methyl)guanidine hydrochloride (Example 37, 800 mg, 2.1 mmol) were added ethanol (15 ml), acetone (4 ml) and concentrated sulfuric acid (134 mg, 1.4 mmol) and the mixture was refluxed for 24 hr. The solvent was evaporated under reduced pressure. Ethanol (11 ml), water (7 ml) and 5N aqueous sodium hydroxide solution (11 ml) were added, and the mixture was refluxed for 2 hr, concentrated under reduced pressure, and extracted with toluene. The solvent was concentrated under reduced pressure, and the residue was purified by silica gel column chromatography (eluent, mixed solution of chloroform:methanol = 10:1) to give the title compound (750 mg) as a gray-white syrupy solid.
¹H-NMR (CDCl₃) δ: 0.86 (3H, t, J=6Hz, CH₃), 1.2-1.6 (16H, m, (CH₂)₈), 1.57 (6H, s, CH₃×2), 3.3-3.4 (2H, m, HNCH₂), 4.89 (4H, s, N(CH₂)₂Ar), 7.2-7.3 (4H, m, ArH), 8.2-9.3 (2H, br,NH).

### Example 13

### 1,6-dihydro-6,6-dimethyl-N⁴-octyl-N²-benzyl-N²-ethyl-1,3,5-triazine-2,4-diamine acetate

To N⁵-octyl-N¹-benzyl-N¹-ethyl-biguanide hydrochloride (Example 38, 1.0 g, 2.7 mmol) were added ethanol (15 ml), acetone (4 ml) and concentrated sulfuric acid (173 mg, 1.8 mmol) and the mixture was refluxed for 24 hr. The solvent was evaporated under reduced pressure. Ethanol (11 ml), water (7 ml) and 5N aqueous sodium hydroxide solution (11 ml) were added, and the mixture was refluxed for 2 hr, concentrated under reduced pressure, and extracted with ethyl acetate. The extract was washed with 5% aqueous sodium acetate solution and water. The solvent was concentrated under reduced pressure, and the residue was purified by silica gel column chromatography (eluent, mixed solution of chloroform:methanol = 10:1) to give the title compound (370 mg) as a pale-yellow syrupy solid.
¹H-NMR (CDCl₃) δ: 0.86 (3H, t, J=6Hz, CH₃), 1.2-1.6 (15H, m, NCH₂CH₃ and (CH₂)₆), 1.46 (6H, s, CH₃×2), 1.94 (3H, s, CH₃COO⁻), 3.2-3.3 (2H, m, NCH₂), 3.5-3.6 (2H, m, NCH₂), 4.71 (2H, s, NCH₂Ar), 7.2-7.4 (5H, m, ArH), 9.70 (1H, br.s, NH), 10.80 (1H, br.s, NH).

### Example 14

### 1,6-dihydro-6,6-dimethyl-N⁴-octyl-N²-methyl-N²-(2-phenylethyl)-1,3,5-triazine-2,4-diamine

To N⁵-octyl-N¹-methyl-N¹-(2-phenylethyl)-biguanide hydrochloride (Example 39, 1.0 g, 2.7 mmol) were added ethanol (15 ml), acetone (4 ml) and concentrated sulfuric acid (173 mg, 1.8 mmol) and the mixture was refluxed for 24 hr. The solvent was evaporated under reduced pressure. Ethanol (11 ml), water (7 ml) and 5N aqueous sodium hydroxide solution (11 ml) were added, and the mixture was refluxed for 2 hr, concentrated under reduced pressure, and extracted with toluene. The solvent was concentrated under reduced pressure, and the residue was purified by silica gel column chromatography (eluent, mixed solution of chloroform:methanol = 10:1) to give the title compound (860 mg) as a pale-yellow solid.
¹H-NMR (CDCl₃) δ: 0.86 (3H, t, J=6Hz, CH₃), 1.2-1.6 (12H, m, (CH₂)₆), 1. 46 (6H, s, CH₃×2), 2.87 (2H, t, J=6Hz, NCH₂CH₂Ar), 3.02 (3H, s, NCH₃), 3.2-3.3 (2H, m, HNCH₂), 3.71 (2H, t, J=6Hz, NCH₂CH₂Ar), 7.1-7.4 (5H, m, ArH), 8.30 (1H, br, NH), 9.20 (1H, br, NH).

### Example 15

### 1,6-dihydro-6,6-dimethyl-N⁴-octyl-N²-methyl-N²-(2-phenylethyl)-1,3,5-triazine-2,4-diamine carbonate

1,6-Dihydro-6,6-dimethyl-N⁴-octyl-N²-methyl-N²-(2-phenylethyl)-1,3,5-triazine (Example 14, 1.8 g, 4.9 mmol) was dissolved in toluene added with a small amount of methanol. Under cooling with acetone-dry ice, carbon dioxide gas was blown in, and the precipitated crystals were collected by filtration to give the title compound (1.4 g) as pale-yellow crystals.
melting point: 72-74°C
¹H-NMR(CDCl₃) δ: 0.86 (3H, t, J=6Hz, CH₃), 1.2-1.6 (12H, m, (CH₂)₆), 1.36 (6H, s, CH₃×2), 2.84 (2H, t, J=8Hz, NCH₂CH₂Ar), 2.97 (3H, s, NCH₃), 3.21 (2H, t, J=8Hz, HNCH₂), 3.63 (2H, t, J=8Hz, NCH₂CH₂Ar), 7.1-7.4 (5H, m, ArH).

### Example 16

### 1,6-dihydro-6,6-dimethyl-N⁴-decyl-N²-methyl-N²-propyl-1,3,5-triazine-2,4-diamine

To N⁵-decyl-N¹-methyl-N¹-propyl-biguanide hydrochloride (Example 40, 800 mg, 2.4 mmol) were added ethanol (15 ml), acetone (4 ml) and concentrated sulfuric acid (153 mg, 1.6 mmol), and the mixture was refluxed for 24 hr. The solvent was evaporated under reduced pressure. Ethanol (11 ml), water (7 ml) and 5N aqueous sodium hydroxide solution (11 ml) were added, and the mixture was refluxed for 2 hr, concentrated under reduced pressure, and extracted with toluene. The solvent was concentrated under reduced pressure, and the residue was purified by silica gel column chromatography (eluent, mixed solution of chloroform:methanol = 10:1) to give the title compound (550 mg) as a brown syrupy solid.
¹H-NMR (CDCl₃) δ: 0.8-1.0 (6H, m, CH₃×2), 1.2-1.7 (18H, m, CH₂ and (CH₂)₈), 1.51 (6H, s, CH₃×2), 3.08 (3H, s, NCH₃), 3.2-3.3 (2H, m, NCH₂), 3.4-3.5 (2H, m, NCH₂), 7.7-8.8 (2H, br, NH).

### Example 17

### 1,6-dihydro-6,6-dimethyl-N⁴-undecyl-N²-(4-aminobenzyl)-N²-methyl-1,3,5-triazine-2,4-diamine dihydrochloride

To N⁵-undecyl-N¹-methyl-N¹-(4-nitrobenzyl)-biguanide dihydrochloride (Example 41, 2.8 g, 5.9 mmol) were added ethanol (40 ml), acetone (11 ml) and concentrated sulfuric acid (91.5 mg, 0.9 mmol) and the mixture was refluxed for 17 hr. The solvent was evaporated under reduced pressure. Ethanol (40 ml), water (8 ml), reduced iron (1.6 g, 28.6 mmol) and calcium chloride (460 mg, 4.1 mmol) were added, and the mixture was refluxed for 3 hr. After celite filtration, the solvent of the filtrate was evaporated under reduced pressure, and the residue was purified by silica gel column chromatography (eluent, mixed solution of chloroform:methanol = 10:1) to give the title compound (300 mg) as a brown syrupy solid.
¹H-NMR (DMSO-d₆) δ: 0.86 (3H, t, J=6Hz, CH₃), 1.2-1.6 (18H, m, (CH₂)₉), 1.44 (6H, s, CH₃×2), 2.93 (3H, s, NCH₃), 3.3-3.5 (2H, m, HNCH₂), 4.58 (2H, s, NCH₂Ar), 6.2 (1H, br, NH₂), 6.64 (2H, d, J=8Hz, ArH), 6.99 (2H, d, J=8Hz, ArH), 7.7-8.8 (4H, br, NH and NH⁺).

### Example 18

### N⁴-tetradecyl-N⁴-methyl-1,6-dihydro-6,6-dimethyl-1, 3, 5-triazine-2,4-diamine hydrochloride

To N⁵-tetradecyl-N⁵-methyl-biguanide hydrochloride (Example 42, 800 mg, 2.3 mmol) were added DMF (10 ml), acetone (3 ml) and camphorsulfonic acid (53.4 mg, 0.23 mmol) and the mixture was refluxed for 18 hr. The solvent was evaporated under reduced pressure. Ethyl acetate was added, the insoluble material was filtered off, and the solvent of the filtrate was evaporated under reduced pressure. The residue was purified by silica gel column chromatography (eluent, mixed solution of chloroform:methanol = 10:1) to give the title compound (400 mg) as pale-yellow crystals.
melting point: 68-70°C
¹H-NMR (DMSO-d₆) δ: 0.85 (3H, t, J=6Hz, CH₃), 1.2-1.6 (24H, m, (CH₂)₁₂), 1.41 (6H, s, CH₃×2), 2.98 (3H, s, NCH₃), 3.4-3.6 (2H, m, HNCH₂), 7.2 (2H, br, NH₂), 7.98 (1H, s, NH), 8.59 (1H, s, NH⁺).

### Example 19

### N⁴-tridecyl-N⁴-methyl-1,6-dihydro-6,6-dimethyl-1,3,5-triazine-2,4-diamine hydrochloride

To N⁵-tridecyl-N⁵-methyl-biguanide hydrochloride (Example 43, 1.0 g, 3.0 mmol) were added DMF (10 ml), acetone (4 ml) and camphorsulfonic acid (70 mg, 0.3 mmol) and the mixture was refluxed for 18 hr. The solvent was evaporated under reduced pressure, and ethyl acetate was added. The insoluble material was filtered off, and the solvent of the filtrate was evaporated under reduced pressure. The residue was purified by silica gel column chromatography (eluent, mixed solution of chloroform:methanol = 10:1) to give the title compound (1.22 g) as pale-yellow crystals.
melting point: 66-68°C
¹H-NMR (CDC1₃) δ: 0.88 (3H, t, J=6Hz, CH₃), 1.2-1.6 (22H, m, (CH₂)₁₁), 1.64 (6H, s, CH₃×2), 3.16 (3H, br.s, NCH₃), 3.4-3.6 (2H, br, NCH₂), 8.02 (1H, br.s, NH), 8.99 (1H, br.s, NH⁺).

### Example 20

### N⁴-dodecyl-N⁴-methyl-1,6-dihydro-6,6-dimethyl-1,3,5-triazine-2,4-diamine hydrochloride

To N⁵-dodecyl-N⁵-methyl-biguanide hydrochloride (Example 44, 1.0 g, 3.1 mmol) were added DMF (10 ml), acetone (4 ml) and camphorsulfonic acid (73 mg, 0.3 mmol) and the mixture was refluxed for 18 hr. The solvent was evaporated under reduced pressure, ethyl acetate was added, and the insoluble material was filtered off. The solvent of the filtrate was evaporated under reduced pressure, and the residue was purified by silica gel column chromatography (eluent, mixed solution of chloroform:methanol = 10:1) to give the title compound (1.14 g) as pale-yellow crystals.
melting point: 59-61°C
¹H-NMR (CDCl₃) δ: 0.88 (3H, t, J=6Hz, CH₃), 1.2-1.6 (20H, m, (CH₂)₁₀), 1.64 (6H, s, CH₃×2), 3.16 (3H, br.s, NCH₃), 3.4-3.6 (2H, br, HNCH₂), 8.0-8.1 (1H, br, NH), 8.98 (1H, br.s, NH⁺).

### Example 21

### N-undecyl-6,6-dimethyl-4-(morpholin-4-yl)-1,6-dihydro-1,3,5-triazine-2-amine acetate

To N-undecyl-N'-(imino-morpholin-4-yl-methyl)guanidine hydrochloride (Example 45, 700 mg, 1.9 mmol) were added ethanol (10 ml), acetone (3 ml) and concentrated sulfuric acid (123 mg, 1.3 mmol) and the mixture was refluxed for 20 hr. The solvent was evaporated under reduced pressure. Ethanol (5 ml), water (5 ml) and 48% aqueous sodium hydroxide solution (1 g) were added, and the mixture was concentrated under reduced pressure. The precipitated crystals were collected by filtration and washed with water to give N-undecyl-6,6-dimethyl-4-(morpholin-4-yl)-1,6-dihydro-1,3,5-triazine-2-amine (638 mg) as colorless crystals. 300 mg therefrom was dissolved in methanol, acetic acid (74 mg, 1.2 mmol) was added, and the solvent was evaporated under reduced pressure. Excess acetic acid was evaporated by 3 repeats of azeotropic distillation with toluene. After cooling, the precipitated crystals were washed with hexane, collected by filtration and dried to give the title compound (330 mg) as colorless crystals.
melting point: 120-124°C
¹H-NMR(CDCl₃) δ: 0.88 (3H, t, J=6Hz, CH₃), 1.2-1.6 (18H, m, (CH₂)₉), 1.46 (6H, s, CH₃×2), 1.95 (3H, s, CH₃COO⁻), 3.2-3.3 (2H, m, HNCH₂), 3.72 (8H, s, NCH₂CH₂O×2), 9.15 (2H, br, NH), 10.15 (1H, br, NH⁺).

### Example 22

### N-octyl-6,6-dimethyl-4-(4-phenylpiperazin-1-yl)-1,6-dihydro-1,3,5-triazine-2-amine

To N-actyl-N'-(imino-4-phenylpiperazin-1-yl-methyl)guanidine hydrochloride (Example 46, 700 mg, 1.8 mmol) were added ethanol (10 ml), acetone (3 ml) and concentrated sulfuric acid (113 mg, 1.2 mmol) and the mixture was refluxed for 20 hr. The solvent was evaporated under reduced pressure. Ethanol (5 ml), water (5 ml) and 48% aqueous sodium hydroxide solution (1 g) were added, and the mixture was refluxed for 2 hr, concentrated under reduced pressure, extracted with toluene and washed with water. The solvent was evaporated under reduced pressure to give the title compound (670 mg) as a pale-yellow syrupy solid.
¹H-NMR(CDCl₃) δ: 0.86 (3H, t, J=6Hz, CH₃), 1.2-1.6 (12H, m, (CH₂)₆), 1.46 (6H, s, CH₃×2), 3.1-3.3 (6H, m, HNCH₂ and NCH₂CH₂N×2), 3.7-3.9 (4H, m, NCH₂CH₂N×2), 6.8-7.0 (3H, m, ArH), 7.2-7.4 (2H, m, ArH).

### Example 23

### 1,6-dihydro-6,6-dimethyl-N⁴-octyl-N²-cyclohexyl-N²-methyl-1,3,5-triazine-2,4-diamine

To N⁵-octyl-N¹-cyclohexyl-N¹-methyl-biguanide hydrochloride (Example 47, 500 mg, 1.5 mmol) were added ethanol (10 ml), acetone (3 ml) and concentrated sulfuric acid (92 mg, 0.9 mmol) and the mixture was refluxed for 20 hr. The solvent was evaporated under reduced pressure. Ethanol (5 ml), water (5 ml) and 48% aqueous sodium hydroxide solution (1 g) were added. The mixture was refluxed for 2 hr, concentrated under reduced pressure, extracted with toluene and washed with water. The solvent was evaporated under reduced pressure to give the title compound (500 mg) as a pale-yellow syrupy solid.
¹H-NMR(CDCl₃) δ: 0.87 (3H, t, J=6Hz, CH₃), 1.2-1.9 (22H, m, (CH₂)₅ and (CH₂)₆), 1.37 (6H, s, CH₃×2), 2.79 (3H, s, NCH₃), 3.19 (2H, t, J=7Hz, HNCH₂), 4.15 (1H, br, NCH).

### Example 24

### N-tridecyl-6,6-dimethyl-4-(morpholin-4-yl)-1,6-dihydro-1,3,5-triazine-2-amine acetate

To N-tridecyl-N'-(imino-morpholin-4-yl-methyl)guanidine hydrochloride (Example 48, 1.0 g, 2.6 mmol) were added ethanol (20 ml), acetone (5 ml) and concentrated sulfuric acid (163 mg, 1.7 mmol) and the mixture was refluxed for 20 hr. The solvent was evaporated under reduced pressure. Ethanol (5 ml), water (5 ml) and 48% aqueous sodium hydroxide solution (1 g) were added, and the mixture was refluxed for 2 hr, and concentrated under reduced pressure. The precipitated crystals were collected by filtration and washed with water to give N-tridecyl-6,6-dimethyl-4-(morpholin-4-yl)-1,6-dihydro-1,3,5-triazine-2-amine (838 mg) as colorless crystals. 500 mg therefrom was dissolved in methanol, acetic acid (114 mg, 1.9 mmol) was added, and the solvent was evaporated under reduced pressure. Excess acetic acid was evaporated by 3 repeats of azeotropic distillation with toluene. After cooling, the precipitated crystals were washed with hexane:ethyl acetate = 1:1, collected by filtration and dried to give the title compound (91 mg) as colorless crystals.
melting point: 118-122°C.
¹H-NMR(CDCl₃) δ: 0.88 (3H, t, J=6Hz, CH₃), 1.2-1.8 (22H, m, (CH₂)₁₁), 1.47 (6H, s, CH₃×2), 1.95 (3H, s, CH₃COO⁻), 3.2-3.3 (2H, m, HNCH₂), 3.72 (8H, s, NCH₂CH₂O×2), 8.8-9.4 (2H, br, NH), 10.4 (1H, br, NH⁺) .

### Example 25

### 1,6-dihydro-6,6-dimethyl-N⁴-dodecyl-N²-(2-methoxyethyl)-N²-methyl-1,3,5-triazine-2,4-diamine

To N⁵-dodecyl-N¹-(2-methoxyethyl)-N¹-methyl-biguanide hydrochloride (Example 49, 850 mg, 2.3 mmol) were added ethanol (20 ml), acetone (5 ml) and concentrated sulfuric acid (143 mg, 1.5 mmol) and the mixture was refluxed for 20 hr. The solvent was evaporated under reduced pressure. Ethanol (5 ml), water (5 ml) and 48% aqueous sodium hydroxide solution (1 g) were added. The mixture was refluxed for 2 hr, concentrated under reduced pressure, extracted with toluene and washed with water. The solvent was evaporated under reduced pressure to give the title compound (890 mg) as a pale-yellow solid.
¹H-NMR(CDCl₃) δ: 0.88 (3H, t, J=6Hz, CH₃), 1.2-1.6 (20H, m, (CH₂)₁₀), 1.34 (6H, s, CH₃×2), 3.00 (3H, s, NCH₃), 3.20 (2H, t, J=7Hz, HNCH₂), 3.36 (3H, s, OCH₃), 3.4-3.6 (4H, m, NCH₂CH₂O).

### Example 26

### N-decyl-6,6-dimethyl-4-(thiomorpholin-4-yl)-1,6-dihydro-1,3,5-triazine-2-amine

To N-decyl-N'-(imino-thiomorpholin-4-yl-methyl)guanidine hydrochloride (Example 50, 1.0 g, 2.6 mmol) were added ethanol (20 ml), acetone (5 ml) and concentrated sulfuric acid (175 mg, 1.8 mmol) and the mixture was refluxed for 20 hr. The solvent was evaporated under reduced pressure. Ethanol (5 ml), water (5 ml) and 48% aqueous sodium hydroxide solution (1 g) were added, and the mixture was refluxed for 2 hr, concentrated under reduced pressure, extracted with toluene and washed with water. The solvent was evaporated under reduced pressure to give the title compound (923 mg) as a colorless solid.
¹H-NMR(CDCl₃) δ: 0.88 (3H, t, J=6Hz, CH₃), 1.2-1.6 (16H, m, (CH₂)₈), 1.33 (6H, s, CH₃×2), 2.6-2.7 (4H, m, NCH₂CH₂S×2), 3.20 (2H, t, J=7Hz, HNCH₂), 3.8-3.9 (4H, m, NCH₂CH₂S×2).

### Example 27

### N⁵-octyl-N⁵-methyl-N¹-(4-chlorobenzyl)biguanide hydrochloride

N¹-octyl-N¹-methyl-cyanoguanidine (Reference Example 1, 10.0 g, 47.5 mmol) and 4-chlorobenzylamine hydrochloride (9.3 g, 52.3 mmol) were suspended in a mixed solution of xylene and toluene (3:1, 320 ml); and the suspension was refluxed for 6 hr, and ice-cooled. The precipitated crystals were collected by filtration and dried. The obtained crystals (16.6 g) were dissolved in 70% aqueous acetonitrile solution (50 ml) and concentrated hydrochloric acid (5 ml) was added. The mixture was concentrated under reduced pressure to a 1/2 volume, and ice-cooled. The precipitated crystals were collected by filtration, washed with ethyl acetate and dried to give crystals (12.1 g). A part of the crystals was recrystallized from water to give the title compound as colorless crystals. melting point: 212-214°C
¹H-NMR(CDCl₃-D₂O) δ: 0.88 ((3H, t, J=6Hz, CH₃), 1.0-1.5 (12H, m, (CH₂)₆), 2.94 (3H, s, CH₃NCH₂), 3.20 (2H, t, J=7Hz, CH₃NCH₂), 4.34 (2H, s, ArCH₂NH), 7.21 (4H, s, ArH).

### Example 28

### N⁵-octyl-N⁵-methyl-N¹-(3,4-dichlorobenzyl)biguanide hydrochloride

N¹-octyl-N¹-methyl-cyanoguanidine (Reference Example 1, 10.0 g, 47.5 mmol) and 3,4-dichlorobenzylamine (8.8 g, 49.9 mmol) were suspended in a mixed solution of xylene and toluene (mixing ratio, 3:1, 320 ml). Concentrated hydrochloric acid (4.6 ml) was added, Dean Stark (azeotropic dehydration apparatus) was set, and the mixture was refluxed for 8 hr, concentrated and ice-cooled. The precipitated crystals were recrystallized from water to give the title compound (12.8 g) as colorless crystals.
melting point: 194-196°C
¹H-NMR(CDCl₃-D₂O) δ: 0.87 ((3H, t, J=6Hz, CH₃), 1.0-1.5 (12H, m, (CH₂)₆), 2.95 (3H, s, NCH₃), 3.19 (2H, brt-like, CH₃NCH₂), 4.32 (2H, s, ArCH₂NH), 7.12 (H, d, d, J=2, 8Hz, ArH), 7.32 (H, d, J=8Hz, ArH), 7.34 (H, s, ArH).

### Example 29

### N⁵-decyl-N¹-methyl-N¹-benzyl-biguanide dihydrochloride

N¹-benzyl-N¹-methyl-cyanoguanidine (Reference Example 2, 13.0 g, 69.1 mmol) and decylamine hydrochloride (14.7 g, 75.9 mmol) were suspended in a mixed solution of xylene and toluene (mixing ratio, 3:1, 240 ml), and the suspension was refluxed for 19 hr. The solvent was evaporated under reduced pressure and the residue was dissolved in 70% aqueous acetonitrile solution (50 ml). Under ice-cooling, concentrated hydrochloric acid (11.5 ml) was added, and the precipitated crystals were recrystallized from 70% aqueous acetonitrile solution to give the title compound (12.7 g) as colorless crystals.
melting point: 150-153°C
¹H-NMR(DMSO-d₆) δ: 0.85 (3H, t, J=6Hz, CH₃), 1.1-1.6 (16H, m, (CH₂)₈), 2.93 (3H, s, NCH₃), 3.11 (2H, m, NHCH₂), 4.64 (2H, s, ArCH₂NH), 4.9-5.5 (4H, br.m, NHx4), 7.2-7.4 (5H, m, ArH).

### Example 30

### N⁵-nonyl-N¹-methyl-N¹-benzyl-biguanide dihydrochloride

N¹-benzyl-N¹-methyl-cyanoguanidine (Reference Example 2, 13.0 g, 69.1 mmol) and nonylamine (10.9 g, 76.1 mmol) were suspended in a mixed solution of xylene and toluene (mixing ratio, 3:1, 240 ml). Concentrated hydrochloric acid (7.0 ml) was added, Dean Stark (azeotropic dehydration apparatus) was set, and the mixture was refluxed for 9 hr. The solvent was evaporated under reduced pressure and the residue was dissolved in 70% aqueous acetonitrile solution (50 ml), and concentrated hydrochloric acid (11.5 ml) was added under ice-cooling. The precipitated crystals were collected by filtration and dried to give the title compound (11.9 g) as colorless crystals.
melting point: 185-187°C
¹H-NMR(DMSO-d₆) δ: 0.85 (3H, t, J=6Hz, CH₃), 1.1-1.5 (14H, m, (CH₂)₇), 2.90 (3H, s, NCH₃), 3.08 (2H, brdt-like, NHCH₂), 4.0-4.6 (H, brm, NH), 4.61 (2H, s, ArCH₂N), 6.6-7.7 (9H, br.m, ArH and NH, NH⁺).

### Example 31

### N⁵-octyl-N¹-benzyl-N¹-methyl-biguanide dihydrochloride

N¹-octyl-cyanoguanidine (15.0 g, 76.4 mmol) and N-methylbenzylamine hydrochloride (13.2 g, 83.8 mmol) were suspended in a mixed solution of xylene and toluene (mixing ratio, 3:1, 360 ml), and the suspension was refluxed for 8 hr. The solvent was evaporated under reduced pressure and the residue was dissolved in 70% aqueous acetonitrile solution (75 ml). Under ice-cooling, concentrated hydrochloric acid (12.8 ml) was added, and the precipitated crystals were collected by filtration and dried to give the title compound (17.4 g) as colorless crystals.
melting point: 168-170°C.
¹H-NMR(DMSO-d₆) δ: 0.85 (3H, t, J=6Hz, CH₃), 1.1-1.6 (12H, m, (CH₂)₆), 2.94 (3H, s, NCH₃) , 3.13 (2H, m, NHCH₂), 4.65 (2H, s, ArCH₂N), 6.0-6.8 (3H, br.m, NH), 7.2-7.4 (5H, m, ArH), 7.6-8.7 (3H, br.m, NH and NH⁺).

### Example 32

### N⁵-octyl-N⁵-methyl-N¹-benzyl-N¹-methyl-biguanide dihydrochloride

N¹-benzyl-N¹-methyl-cyanoguanidine (Reference Example 2, 13.0 g, 69.1 mmol) and N-methyloctylamine (10.4 g, 72.6 mmol) were suspended in a mixed solution of xylene and toluene (mixing ratio, 3:1, 240 ml). Concentrated hydrochloric acid (6.6 ml) was added, Dean Stark (azeotropic dehydration apparatus) was set, and the mixture was refluxed for 8 hr and ice-cooled. The precipitated crystals were collected by filtration and dried. The obtained crystals (18.5 g) were dissolved in 70% aqueous acetonitrile solution (50 ml), concentrated hydrochloric acid (8.4 ml) was added and the mixture-was ice-cooled. The precipitated crystals were collected by filtration, washed with ethyl acetate, and dried to give the title compound (14.3 g) as colorless crystals.
melting point: 203-205°C
¹H-NMR(DMSO-d₆) δ: 0.84 (3H, t, J=6Hz, CH₃), 1.1-1.6 (12H, m, (CH₂)₆), 2.90, 2.93 (each 3H, s, NCH₃×2), 3.28 (2H, t, J=7Hz, CH₃NCH₂), 4.60 (2H, s, ArCH₂N), 6.6-7.4 (5H, br.m, NH and NH⁺), 7.2-7.4 (5H, m, ArH).

### Example 33

### N⁵-octyl-N⁵-methyl-N¹-(4-methylbenzyl)biguanide hydrochloride

N¹-octyl-N¹-methyl-cyanoguanidine (Reference Example 1, 10.0 g, 47.5 mmol) and 4-methylbenzylamine hydrochloride (7.9 g, 50.1 mmol) were suspended in a mixed solution of xylene and toluene (mixing ratio, 3:1, 320 ml), and the suspension was refluxed for 9 hr and ice-cooled. The precipitated crystals were collected by filtration, washed with toluene and dried to give the title compound (14.0 g) as colorless crystals.
melting point: 194-197°C
¹H-NMR(CDCl₃) δ: 0.87 (3H, t, J=6Hz, CH₃), 1.0-1.5 (12H, m, (CH₂)₆), 2.28 (3H, s, ArCH₃), 2.97 (3H, s, CH₃NCH₂), 3.25 (2H, t, J=7Hz, CH₃NCH₂), 4.35 (2H, d, J=6Hz, ArCH₂NH), 6.69, 6.77, 7.66 (5H, br.s, NH, NH⁺), 7.05 (2H, d, J=8Hz, ArH), 7.16 (2H, d, J=8Hz, ArH).

### Example 34

### N-dodecyl-N'-(imino-morpholin-4-yl-methyl)guanidine hydrochloride

N¹-dodecyl-cyanoguanidine (1.5 g, 5.9 mmol) and morpholine (520 mg, 5.9 mmol) were suspended in xylene (10 ml). Concentrated hydrochloric acid (0.62 g, 5.9 mmol) was added, Dean Stark (azeotropic dehydration apparatus) was set, and the mixture was refluxed for 6 hr and cooled. Ethyl acetate was added, and the precipitated crystals were collected by filtration and dried to give the title compound (2.0 g) as a colorless solid.
¹H-NMR(DMSO-d₆) δ: 0.88 (3H, t, J=6Hz, CH₃), 1.2-1.5 (20H, m, (CH₂)₁₀), 3.0-3.1 (2H, m, HNCH₂), 3.4-3.5 (4H, m, NCH₂CH₂O×2), 3.6-3.7 (4H, m, NCH₂CH₂O×2). 6.8-7.6 (5H, br, NH).

### Example 35

### N-dodecyl-N'-(imino-4-methylpiperazin-1-yl-methyl)guanidine hydrochloride

N¹-dodecyl-cyanoguanidine (2.5 g, 10.0 mmol) and methylpiperazine (1.0 g, 10.0 mmol) were suspended in xylene (12 ml). Concentrated hydrochloric acid (1.0 g, 10.0 mmol) was added, Dean Stark (azeotropic dehydration apparatus) was set, and the mixture was refluxed for 6 hr and cooled. Ethyl acetate was added, and the precipitated crystals were collected by filtration and dried to give the title compound (2.8 g) as colorless crystals.
melting point: 151-152°C
¹H-NMR(DMSO-d₆) δ: 0.86 (3H, t, J=6Hz, CH₃), 1.2-1.5 (20H, m, (CH₂)₁₀), 2.19 (3H, s, NCH₃), 2.3-2.4 (4H, m, NCH₂CH₂N×2), 3.0-3.1 (2H, m, HNCH₂), 3.4-3.5 (4H, m, NCH₂CH₂N×2), 6.7-7.5 (5H, br, NH and NH⁺).

### Example 36

### N-decyl-N'-(imino-piperidin-1-yl-methyl)guanidine hydrochloride

N¹-decyl-cyanoguanidine (1.4 g, 6.0 mmol) and piperidine (510 mg, 6.0 mmol) were suspended in xylene (10 ml). Concentrated hydrochloric acid (620 mg, 6.0 mmol) was added, Dean Stark (azeotropic dehydration apparatus) was set, and the mixture was refluxed for 6 hr and cooled. Ethyl acetate was added, and the precipitated crystals were collected by filtration and dried to give the title compound (1.2 g) as colorless crystals.
melting point: 162-164°C
¹H-NMR(DMSO-d₆) δ: 0.88 (3H, t, J=6Hz, CH₃), 1.2-1.7 (22H, m, N(CH₂)₂(CH₂)₃ and (CH₂)₈), 2.9-3.1 (2H, m, HNCH₂), 3.4-3.5 (4H, br, N(CH₂)₂(CH₂)₃), 6.6-7.4 (5H, br, NH and NH⁺).

### Example 37

### N-decyl-N'-(imino-1,3-dihydro-2H-isoindol-2-yl-methyl)guanidine hydrochloride

N¹-decyl-cyanoguanidine (940 mg, 4.2 mmol) and 1,3-dihydro-2H-isoindole (500 mg, 4.2 mmol) were suspended in xylene (8 ml). Concentrated hydrochloric acid (440 mg, 4.2 mmol) was added, Dean Stark (azeotropic dehydration apparatus) was set, and the mixture was refluxed for 6 hr and cooled. Ethyl acetate was added, and the precipitated crystals were collected by filtration and dried to give the title compound (886 mg) as a gray solid.
¹H-NMR(DMSO-d₆) δ: 0.88 (3H, t, J=6Hz, CH₃), 1.2-1.6 (16H, m, (CH₂)₈), 3.0-3.2 (2H, m, HNCH₂), 4.71 (4H, s, N(CH₂)₂Ar), 6.8-7.6 (5H, br, NH and NH⁺), 7.3-7.5 (4H, m, ArH).

### Example 38

### N⁵-octyl-N¹-benzyl-N¹-ethyl-biguanide hydrochloride

N¹-octyl-cyanoguanidine (1.5 g, 7.6 mmol) and benzylethylamine (1.0 g, 7.6 mmol) were suspended in xylene (10 ml). Concentrated hydrochloric acid (800 mg, 7.6 mmol) was added, Dean Stark (azeotropic dehydration apparatus) was set, and the mixture was refluxed for 6 hr and cooled. Ethyl acetate was added, and the precipitated crystals were collected by filtration and dried to give the title compound (2.24 g) as a colorless solid.
¹H-NMR(DMSO-d₆) δ: 0.88 (3H, t, J=6Hz, CH₃), 1.04 (3H, t, J=6Hz, CH₃), 1.2-1.5 (12H, m, (CH₂)₆), 3.0-3.1 (2H, m, NCH₂), 3.2-3.3 (2H, m, NCH₂), 4.60 (2H, s, NCH₂Ar), 6.8-7.6 (5H, br, NH and NH⁺), 7.2-7.4 (5H, m, ArH).

### Example 39

### N⁵-octyl-N¹-methyl-N¹-(2-phenylethyl)-biguanide hydrochloride

N¹-octyl-cyanoguanidine (730 mg, 3.7 mmol) and 2-phenylethylamine (500 mg, 3.7 mmol) were suspended in xylene (8 ml). Concentrated hydrochloric acid (390 mg, 3.8 mmol) was added, Dean Stark (azeotropic dehydration apparatus) was set, and the mixture was refluxed for 6 hr and cooled. Ethyl acetate was added, and the precipitated crystals were collected by filtration and dried to give the title compound (1074 mg) as a colorless solid.
¹H-NMR(DMSO-d₆) δ: 0.86 (3H, t, J=6Hz, CH₃), 1.2-1.5 (12H, m, (CH₂)₆), 2.8-2.9 (2H, m, NCH₂CH₂Ar), 2.90 (3H, s, NCH₃) , 3.0-3.1 (2H, m, NCH₂), 3.5-3.6 (2H, m, NCH₂CH₂Ar) , 6.7-7.4 (5H, br, NH and NH⁺), 7.2-7.4 (5H, m, ArH).

### Example 40

### N⁵-decyl-N¹-methyl-N¹-propyl-biguanide hydrochloride

N¹-decyl-cyanoguanidine (1.54 g, 6.9 mmol) and methylpropylamine (500 mg, 6.9 mmol) were suspended in xylene (8 ml). Concentrated hydrochloric acid (720 mg, 6.9 mmol) was added, Dean Stark (azeotropic dehydration apparatus) was set, and the mixture was refluxed for 6 hr and cooled. Ethyl acetate was added, and the precipitated crystals were collected by filtration and dried to give the title compound (850 mg) as a colorless solid.
¹H-NMR(DMSO-d₆) δ : 0.8-0.9 (6H, m, CH₃×2), 1.2-1.6 (18H, m, CH₂ and (CH₂)₈), 2.92 (3H, s, NCH₃), 3.0-3.1 (2H, m, NCH₂), 3.27 (2H, t, J=7Hz, NCH₂), 6.6-7.3 (5H, br, NH and NH⁺).

### Example 41

### N⁵-undecyl-N¹-methyl-N¹-(4-nitrobenzyl)-biguanide dihydrochloride

N¹-undecyl-cyanoguanidine (3.3 g, 13.8 mmol) and methyl-p-nitrobenzylamine (2.3 g, 13.8 mmol) were suspended in xylene (8 ml). Concentrated hydrochloric acid (1.44 g, 13.8 mmol) was added, Dean Stark (azeotropic dehydration apparatus) was set, and the mixture was refluxed for 6 hr and cooled. Ethyl acetate was added, and the precipitated insoluble material was filtered off. The filtrate was purified by silica gel column chromatography (eluent, mixed solution of chloroform:methanol = 10:1) to give the title compound (5.6 g) as a yellow syrupy solid.
¹H-NMR (CD₃OD) δ: 0.90 (3H, br, CH₃), 1.2-1.8 (18H, m, (CH₂)₉), 3.17 (3H, s, NCH₃), 4.93 (2H, s, NCH₂Ar), 7.66 (2H, d, J=8Hz, ArH), 8.31 (2H, d, J=8Hz, ArH).

### Example 42

### N⁵-tetradecyl-N⁵-methyl-biguanide hydrochloride

Methyltetradecylamine hydrochloride (2.04 g, 7.7 mmol) and dicyandiamide (650 mg, 7.7 mmol) were stirred at an outer temperature of 170 to 180°C for 1 hr, and allowed to cool to room temperature. Ethanol was added, and the mixture was dissolved. The insoluble material was filtered off, and the filtrate was concentrated under reduced pressure. Ethyl acetate was added, and the precipitated crystals were collected by filtration, washed with ethyl acetate, and dried to give the title compound (1.60 g) as a colorless solid. ¹H-NMR(DMSO-d₆) δ : 0.86 ((3H, t, J=6Hz, CH₃), 1.2-1.6 (24H, m, (CH₂)₁₂), 2.90 (3H, s, NCH₃), 3.29 (2H, t, J=8Hz, NCH₂), 6.70 (4H, s, NH and NH₂), 7.19 (2H, s, NH and NH⁺).

### Example 43

### N⁵-tridecyl-N⁵-methyl-biguanide hydrochloride

Methyltridecylamine hydrochloride (2.78 g, 11.1 mmol) and dicyandiamide (933 mg, 11.1 mmol) were stirred at an outer temperature of 170 to 180°C for 1 hr, and allowed to cool to room temperature. Ethanol was added, and the mixture was dissolved. The insoluble material was filtered off, and the filtrate was concentrated under reduced pressure. Ethyl acetate was added, and the precipitated crystals were collected by filtration, washed with ethyl acetate, and dried to give the title compound (2.37g) as a pale-yellow solid.
¹H-NMR(DMSO-d₆) δ: 0.86 (3H, t, J=6Hz, CH₃), 1.2-1.6 (22H, m, (CH₂)₁₁), 2.90 (3H, s, NCH₃), 3.2-3.3 (2H, m, NCH₂), 6.70 (4H, s, NH and NH₂), 7.19 (2H, s, NH and NH⁺).

### Example 44

### N⁵-dodecyl-N⁵-methyl-biguanide hydrochloride

Methyldodecylamine hydrochloride (2.38 g, 10.1 mmol) and dicyandiamide (849 mg, 10.1 mmol) were stirred at an outer temperature of 170 to 180°C for 1 hr, and allowed to cool to room temperature. Ethanol was added, and the mixture was dissolved. The insoluble material was filtered off, and the filtrate was concentrated under reduced pressure. Ethyl acetate was added, and the precipitated crystals were collected by filtration, washed with ethyl acetate, and dried to give the title compound (2.13 g) as a colorless solid.
¹H-NMR(DMSO-d₆) δ: 0.86 (3H, t, J=6Hz, CH₃), 1.2-1.6 (20H, m, (CH₂)₁₀), 2.90 (3H, s, NCH₃), 3.2-3.3 (2H, m, NCH₂), 6.68 (4H, s, NH and NH₂), 7.19 (2H, s, NH and NH⁺).

### Example 45

### N-undecyl-N'-(imino-morpholin-4-yl-methyl)guanidine hydrochloride

N¹-undecyl-cyanoguanidine (1.0 g, 4.2 mmol) and morpholine (366 mg, 4.2 mmol) were suspended in xylene (7 ml). Concentrated hydrochloric acid (440 mg, 4.2 mmol) was added, Dean Stark (azeotropic dehydration apparatus) was set, and the mixture was refluxed for 6 hr and cooled. Ethyl acetate was added, and the precipitated crystals were collected by filtration and dried to give the title compound (1.2 g) as a colorless solid.
¹H-NMR(DMSO-d₆) δ: 0.86 (3H, t, J=7Hz, CH₃), 1.2-1.5 (18H, m, (CH₂)₉), 3.0-3.1 (2H, m, HNCH₂), 3.4-3.5 (4H, m, NCH₂CH₂O×2), 3.6-3.7 (4H, m, NCH₂CH₂O×2), 6.7-7.6 (5H, br, NH and NH⁺).

### Example 46

### N-octyl-N'-(imino-4-phenylpiperazin-1-yl-methyl)guanidine hydrochloride

N¹-octyl-cyanoguanidine (1.0 g, 5.1 mmol) and 4-phenylpiperazine (826 mg, 5.1 mmol) were suspended in xylene (10 ml) and concentrated hydrochloric acid (530 mg, 5.1 mmol) was added. Dean Stark (azeotropic dehydration apparatus) was set, and the mixture was refluxed for 6 hr and cooled. Ethyl acetate was added and the precipitated crystals were collected by filtration and dried to give the title compound (1.4 g) as colorless crystals.
melting point: 161-165°C
¹H-NMR(DMSO-d₆) δ: 0.86 (3H, t, J=6Hz, CH₃), 1.2-1.6 (12H, m, (CH₂)₆), 3.0-3.2 (6H, m, HNCH₂ and NCH₂CH₂N×2), 3.6-3.7 (4H, br, NCH₂CH₂N×2), 6.8-7.5 (5H, br, NH and NH⁺), 6.8-7.3 (5H, m, ArH).

### Example 47

### N⁵-octyl-N¹-cyclohexyl-N¹-methyl-biguanide hydrochloride

Cyclohexylmethylamine hydrochloride (1.0 g, 6.7 mmol) was dissolved in water (0.5 ml). Xylene (15 ml) and N¹-octyl-cyanoguanidine (1.3 g, 6.7 mmol) were added, Dean Stark (azeotropic dehydration apparatus) was set, and the mixture was refluxed for 6 hr and cooled. Ethyl acetate was added, and the precipitated crystals were collected by filtration and dried to give the title compound (730 mg) as colorless crystals.
melting point: 139-143°C
¹H-NMR(DMSO-d₆) δ: 0.86 (3H, t, J=6Hz, CH₃), 1.0-2.0 (22H, m, (CH₂)₅ and (CH₂)₆), 2.76 (3H, s, NCH₃), 3.0-3.1 (2H, m, HNCH₂), 3.9-4.0 (1H, br, NCH), 6.6-7.4 (5H, m, NH and NH⁺).

### Example 48

### N-tridecyl-N'-(imino-morpholin-4-yl-methyl)guanidine hydrochloride

N-[cyano(imino)methyl]morpholine-4-carboxyimidamide (1.89 g, 12.2 mmol) and tridecylamine (2.44 g, 12.2 mmol) were suspended in xylene (20 ml). Concentrated hydrochloric acid (1.28 g, 12.2 mmol) was added, Dean Stark (azeotropic dehydration apparatus) was set, and the mixture was refluxed for 6 hr and cooled. Ethyl acetate was added, and the precipitated crystals were collected by filtration and dried to give the title compound (3.79 g) as a colorless solid.
¹H-NMR(DMSO-d₆) δ: 0.86 (3H, t, J=6Hz, CH₃), 1.2-1.5 (22H, m, (CH₂)₁₁), 3.0-3.1 (2H, m, HNCH₂), 3.4-3.5 (4H, m, NCH₂CH₂O×2), 3.6-3.7 (4H, m, NCH₂CH₂O×2), 6.7-8.1 (5H, br, NH and NH⁺).

### Example 49

### N⁵-dodecyl-N¹-(2-methoxyethyl)-N¹-methyl-biguanide hydrochloride

N¹-dodecyl-cyanoguanidine (3.13 g, 11.2 mmol) and 2-methoxyethylamine (1.0 g, 11.2 mmol) were suspended in xylene (20 ml). Concentrated hydrochloric acid (1.17 g, 11.2 mmol) was added, Dean Stark (azeotropic dehydration apparatus) was set, and the mixture was refluxed for 6 hr and cooled. The reaction solution was purified by silica gel column chromatography (eluent, chloroform:methanol = 10:1) to give the title compound (880 mg) as a colorless solid.
¹H-NMR(DMSO-d₆) δ: 0.86 (3H, t, J=6Hz, CH₃), 1.2-1.5 (20H, m, (CH₂)₁₀), 2.96 (3H, s, NCH₃), 3.0-3.1 (2H, m, HNCH₂), 3.26 (3H, s, OCH₃), 3.4-3.5 (4H, m, NCH₂CH₂O), 6.7-7.4 (5H, br, NH and NH⁺).

### Example 50

### N-decyl-N'-(imino-thiomorpholin-4-yl-methyl)guanidine hydrochloride

N¹-decyl-cyanoguanidine (2.0 g, 8.9 mmol) and thiomorpholine (922 mg, 8.9 mmol) were suspended in xylene (20 ml) and concentrated hydrochloric acid (930 mg, 8.9 mmol) was added. Dean Stark (azeotropic dehydration apparatus) was set, and the mixture was refluxed for 6 hr and cooled. Ethyl acetate was added, and the precipitated crystals were collected by filtration and dried to give the title compound (2.3 g) as a colorless solid.
¹H-NMR(DMSO-d₆) δ: 0.86 (3H, t, J=6Hz, CH₃), 1.2-1.5 (16H, m, (CH₂)₈), 2.6-2.7 (4H, m, NCH₂CH₂S×2), 3.0-3.1 (2H, m, HNCH₂), 3.7-3.8 (4H, m, NCH₂CH₂S×2), 6.8-7.6 (5H, br, NH and NH⁺).

The dihydrotriazine derivatives and biguanide derivatives obtained in the above-mentioned Examples were examined for the bactericidal activity (Experimental Example 1), water solubility (Experimental Example 2), and skin sensitivity (allergenicity) (Experimental Example 3).

### Experimental Example 1

### Bactericidal activity test (bactericidal efficacy evaluation (MBC measurement))

### (1) Preparation of bacteria solution

Each strain was cultured in Trypticase Soy agar medium for 18 to 20 hr, suspended in 0.1% peptone saline, and prepared to OD 600nm, 1.0 McFarland. The suspension was further diluted with 0.1% peptone saline to give a test bacteria solution (targeted value; 2x107 CFU/mL).

### (2) Drug preparation method

The prepared each drug and control (chlorhexidine gluconate) were diluted with sterile purified water to 1000 µg/mL, and 2-fold dilution series (200, 100, 50, 25, 12.5, 6.25, 3.13, 1.56 µg/mL) were prepared.

### (3) Drug reaction and evaluation

A test bacteria solution (10 µL) was added to diluted solutions of respective test preparation and control (190 µL). At 0.5, 1 and 3 min from the start of the reaction, a part of the reaction mixture was sampled and mixed with an equal amount of a neutralizing agent (10% Tween 80, 3% lecithin/aqueous solution) to terminate the bactericidal reaction. The reaction mixture was inoculated to a 96 well microplate dispensed with 180 µL of Tripticase Soy liquid medium in advance, and statically cultured at 37°C for 20 to 24 hr. Thereafter, the presence or absence of growth was visually evaluated, and the minimum concentration at which growth was not observed was taken as the minimal bactericidal concentration (MBC value) of each action time. The test results are shown in Tables 1 to 8. The numerical values in the Tables show MBC, the unit is µg/mL, S. aureus means *Staphylococcus aureus,* MRSA means methicillin-resistant *Staphylococcus aureus,* E.coli means *Escherichia coli,* and P.aeruginosa means *Pseudorcconas aeruginosa.*

**Table 1**

| Strain | Example 1 | | | Example 2 | | | Example 3 | | |
|---|---|---|---|---|---|---|---|---|---|
| | 0.5 min | 1 min | 3 min | 0.5 min | 1 min | 3 min | 0.5 min | 1 min | 3 min |
| S.aureus ATCC 6538 | 50 | 12 | 6.25 | 25 | 25 | 12.5 | 25 | 25 | 12.5 |
| MRSA 33591 ATCC 33591 | 50 | 25 | 12.5 | 25 | 25 | 12.5 | 25 | 25 | 25 |
| E coli ATCC 25922 | 12.5 | 12.5 | 12.5 | 12.5 | 6.25 | 6.25 | 12.5 | 12.5 | 12.5 |
| P.aeruginosa ATCC 27853 | 12.5 | 12.5 | 12.5 | 25 | 25 | 12.5 | 25 | 25 | 25 |
| P.aeruginosa PAO-1 | 12.5 | 12.5 | 12.5 | 25 | 25 | 12.5 | 25 | 25 | 25 |

**Table 2**

| Strain | Example 4 | Example 5 | Example 6 |
|---|---|---|---|
| | 0.5 min | 0.5 min | 0.5 min |
| S.aureus ATCC 6538 | 100 | 200 | >200 |
| MRSA ATCC 33591 | 25 | 50 | 200 |
| E.coli ATCC 25922 | 12.5 | 25 | 25 |
| P.aeruginosa ATCC 27853 | 25 | 12.5 | 25 |
| P.aeruginosa PAO-1 | 25 | 25 | 25 |

**Table 3**

| Strain | Example 8 | | | Example 10 | | |
|---|---|---|---|---|---|---|
| | 0.5 min | 1 min | 3 min | 0.5 min | 1 min | 3 min |
| S.aureus ATCC 6538 | 37.5 | 25 | 9.4 | 75 | 50 | 12.5 |
| MRSA 33591 ATCC 33591 | 37.5 | 18.8 | 6.3 | 50 | 50 | 12.5 |
| E.coli ATCC 25922 | 18.8 | 12.5 | 9.4 | 25 | 25 | 25 |
| P.aeruginosa ATCC 27853 | 12.5 | 12.5 | 9.4 | 25 | 18.8 | 18.8 |

**Table 4**

| Strain | Example 11 | | | Example 12 | | | Example 13 | | |
|---|---|---|---|---|---|---|---|---|---|
| | 0.5 min | 1 min | 3 min | 0.5 min | 1 min | 3 min | 0.5 min | 1 min | 3 min |
| S.aureus ATCC 6538 | 50 | 37.5 | 25 | 25 | 18.8 | 4.7 | 50 | 25 | 18.8 |
| MRSA ATCC 33591 | 75 | 62.5 | 31.3 | 25 | 12.5 | 6.3 | 62.5 | 37.5 | 18.8 |
| E.coli ATCC 25922 | 18.8 | 18.8 | 18.8 | 18.8 | 18.8 | 15.7 | 25 | 18.8 | 12.5 |
| P.aeruginosa ATCC 27853 | 25 | 25 | 18.8 | 37.5 | 37.5 | 18.8 | 18.8 | 18.8 | 18.8 |

**Table 5**

| Strain | Example 14 | | | Example 16 | | | Example 17 | | |
|---|---|---|---|---|---|---|---|---|---|
| | 0.5 min | 1 min | 3 min | 0.5 min | 1 min | 3 min | 0.5 min | 1 min | 3 min |
| S.aureus ATCC 6538 | 75 | 37.5 | 18.8 | 75 | 50 | 25 | 25 | 15.7 | 7.8 |
| MRSA ATCC 33591 | 62.5 | 31.3 | 15.7 | 100 | 37.5 | 18.8 | 18.8 | 12.5 | 6.3 |
| E.coli ATCC 25922 | 25 | 25 | 12.5 | 18.8 | 18.8 | 9.4 | 25 | 18.8 | 12.5 |
| P.aeruginosa ATCC 27853 | 25 | 18.8 | 12.5 | 25 | 18.8 | 18.8 | 37.5 | 18.8 | 9.4 |

**Table 6**

| Strain | Example 18 | | |
|---|---|---|---|
| | 0.5 min | 1 min | 3 min |
| S.aureus ATCC 6538 | 18.8 | 12.5 | 4.7 |
| MRSA ATCC 33591 | 18.8 | 9.4 | 4.7 |
| E.coli ATCC 25922 | 12.5 | 12.5 | 6.3 |
| P.aeruginosa ATCC 27853 | 18.8 | 12.5 | 9.4 |

**Table 7**

| Strain | Example 27 | | | Example 28 | | | Example 29 | | |
|---|---|---|---|---|---|---|---|---|---|
| | 0.5 min | 1 min | 3 min | 0.5 min | 1 min | 3 min | 0.5 min | 1 min | 3 min |
| S.aureus ATCC 6538 | 50 | 25 | 25 | 25 | 12.5 | 12.5 | 200 | 100 | 12.5 |
| MRSA ATCC 33591 | 50 | 25 | 25 | 50 | 12.5 | 12.5 | 200 | 200 | 50 |
| E.coli ATCC 25922 | 25 | 6.25 | 6.25 | 6.25 | 6.25 | 6.25 | 6.25 | 6.25 | 6.25 |
| P.aeruginosa ATCC 27853 | 12.5 | 12.5 | 6.25 | 25 | 25 | 12.5 | 12.5 | 12.5 | 6.25 |
| P.aeruginosa PAO-1 | 12.5 | 12.5 | 12.5 | 12.5 | 12.5 | 6.25 | 12.5 | 12.5 | 6.25 |

**Table 8**

| Strain | Example 30 | | | control | | |
|---|---|---|---|---|---|---|
| | 0.5 min | 1 min | 3 min | 0.5 min | 1 min | 3 min |
| S.aureus ATCC 6538 | 200 | 100 | 25 | >200 | >200 | >200 |
| MRSA ATCC 33591 | >200 | >100 | 25 | >200 | >200 | 200 |
| E.coli ATCC 25922 | 12.5 | 12.5 | 12.5 | 100 | 25 | 25 |
| P.aeruginosa ATCC 27853 | 6.25 | 6.25 | 6.25 | >200 | 12.5 | 25 |
| P.aeruginosa PAO-1 | 12.5 | 6.25 | 6.25 | >200 | 25 | 12.5 |

It is appreciated from the bactericidal efficacy evaluation shown in Table 1 to Table 8 that all compounds of Examples have a high bactericidal activity against various bacterial strains by a contact for a short time.

### Experimental Example 2

### Water solubility test

According to the Japanese Pharmacopoeia solubility test method, the compounds of Examples 1 and 7 of the present invention and the corresponding N-methyl unsubstituted compounds were examined for water solubility. To be specific, a test substance was powderized, placed in water and the mixture was vigorously shaken for 30 sec every 5 min at 20+5°C. The solubility was evaluated based on the degree of dissolution in 30 min by reference to the following Table 9. The results are shown in Table 10.

**Table 9**

| criteria | The amount of solvent necessary for dissolving 1 g of solute | |
|---|---|---|
| very soluble | less than 1 mL | |
| freely soluble | not less than 1 mL | less than 10 mL |
| soluble | not less than 10 mL | less than 30 mL |
| sparingly soluble | not less than 30 mL | less than 100 mL |
| slightly soluble | not less than 100 mL | less than 1,000 mL |
| very slightly soluble | not less than 1,000 mL | less than 10,000 mL |
| practically insoluble | not less than 10,000 mL | |

**Table 10**

| compound | water (mL) to dissolve 1 g | evaluation |
|---|---|---|
| Ex. 1 | <0.5 | very soluble. |
| 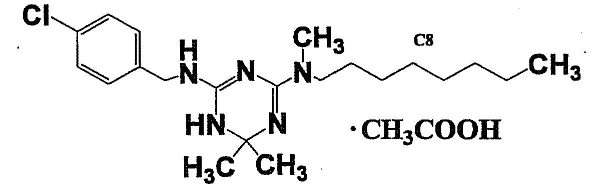 | | |
| Comp. Ex. 1 | 110 | slightly soluble |
| 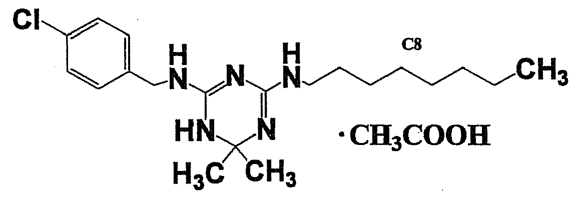 | | |
| Ex. 7 | 0.9 | very soluble |
| 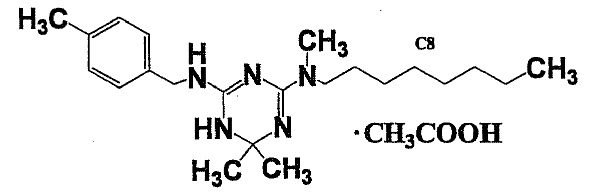 | | |
| comp. Ex. 2 | 50 | sparingly soluble |
| 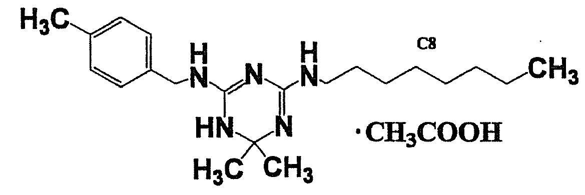 | | |

As is clear from the above results, a drastic improvement in the water solubility was observed by substitution of nitrogen of the amino group by a methyl group.

### Experimental Example 3

### Skin sensitivity test (LLNA-BrdU method)

For application of an external bactericidal/disinfectant agent to the skin, the sensitivity (allergenicity) is desirably as small as possible. It was found that the sensitivity of the compound of the present invention was markedly improved as compared to analogous compounds. The sensitivity was evaluated by a Local lymph node assay (LLNA) method using Bromodeoxyuridine (BrdU). In this test, measurement of the amount of BrdU uptaken by the lymph node using an ELISA kit was not performed, and the evaluation was based solely on the weight increase of the lymph node.

8-Week-old female CBA/JN Crlj mice were grouped (3 per group) according to the adequate strafication method based on the body weight (about 20 g) on the first day of administration. A test substance was prepared to 5 w/v% for each test substance by using a mixed solution of acetone and olive oil (v/v=4:1, AOO). This was repeatedly administered transdermally to the auricle of the mice at 25 µL/auricle (50 µL for both auricles) once a day for 3 days. Two days after the final administration, BrdU 10 mg/mL (0.5 mL/mouse) was intraperitoneally administered. Three days after the final transdermal administration (next day of BrdU administration), carbon dioxide was given by inhalation to a comfortably death, and the auricle lymph node was isolated and the weight thereof was measured. The lymph node weights of each test substance group were averaged, and the level of sensitivity was evaluated based on the ratio to the weight of the control group (solvent group) (Stimulation index: SI value). The results are shown in Table 11. From Table 11, the skin sensitivity of the compound of the present invention was clearly improved as compared to the compounds having a bactericidal/disinfectant effect disclosed in WO2004/054989 (Reference Examples 3, 4 and 5),

**Table 11**

| compound | SI value |
|---|---|
| Ex. 5 | 0.9 |
| 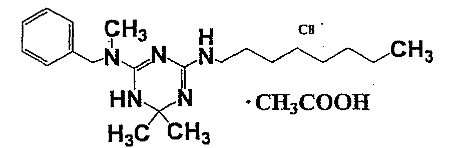 | |
| Ex. 7 | 2.6 |
| 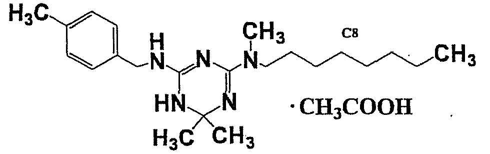 | |
| Ex. 27 | 2.4 |
| 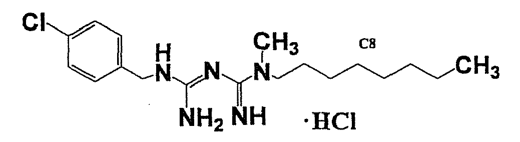 | |
| Ref. Ex. 3 | 4.6 |
| 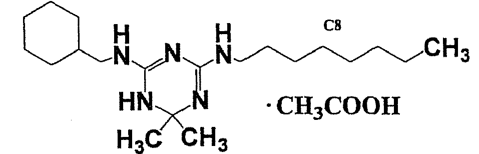 | |
| Ref. Ex. 4 | 4.4 |
| 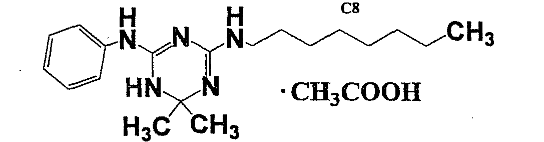 | |
| Ref. Ex. 5 | 4.4 |
| 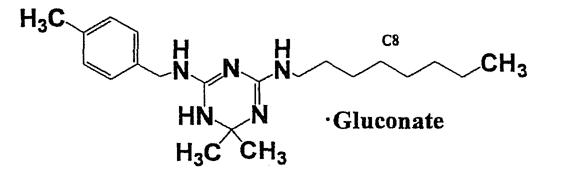 | |

This application is based on a patent application No. 2008-215765 filed in Japan, the contents of which are incorporated in full herein reference.

## Claims

1. A compound represented by the formula (1) 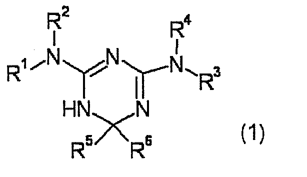 wherein
R¹ is a hydrogen atom, an optionally substituted alkyl group,
an optionally substituted cycloalkyl group, an optionally substituted aryl group, an optionally substituted aralkyl group or an optionally substituted heterocyclic group,
R² is a hydrogen atom or an optionally substituted alkyl group, or R¹ and R² optionally form, together with the adjacent nitrogen atom, an optionally substituted nitrogen-containing heterocyclic group,
R⁴ is a hydrogen atom or an optionally substituted alkyl group,
R³ is an optionally substituted alkyl group, and
R⁵ and R⁶ are the same or different and each is a hydrogen atom or a methyl group,
excluding a compound wherein both R² and R⁴ are hydrogen atoms and a compound wherein both R¹ and R⁴ are hydrogen atoms, or a tautomer thereof or a salt thereof.

2. The compound according to claim 1,
wherein
R¹ is a hydrogen atom, an optionally substituted alkyl group,
an optionally substituted cycloalkyl group, an optionally substituted aryl group, an optionally substituted aralkyl group or an optionally substituted heterocyclic group,
R² and R⁴ are the same or different and each is a hydrogen atom or an optionally substituted alkyl group,
R³ is an optionally substituted alkyl group, and
R⁵ and R⁶ are the same or different and each is a hydrogen atom or a methyl group,
excluding a compound wherein both R² and R⁴ are hydrogen atoms and a compound wherein both R¹ and R⁴ are hydrogen atoms, or a tautomer thereof or a salt thereof.

3. A compound represented by the formula (2) 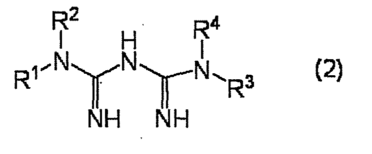 wherein
R¹ is a hydrogen atom, an optionally substituted alkyl group, an optionally substituted cycloalkyl group, an optionally substituted aryl group, an optionally substituted aralkyl group or an optionally substituted heterocyclic group,
R² is a hydrogen atom or an optionally substituted alkyl group, or R¹ and R² optionally form, together with the adjacent nitrogen atom, an optionally substituted nitrogen-containing heterocyclic group,
R⁴ is a hydrogen atom or an optionally substituted alkyl group, and
R³ is an optionally substituted alkyl group,
excluding a compound wherein both R² and R⁴ are hydrogen atoms and a compound wherein both R¹ and R⁴ are hydrogen atoms, or a tautomer thereof or a salt thereof.

4. The compound according to claim 3,
wherein
R¹ is a hydrogen atom, an optionally substituted alkyl group, an optionally substituted cycloalkyl group, an optionally substituted aryl group, an optionally substituted aralkyl group or an optionally substituted heterocyclic group,
R² and R⁴ are the same or different and each is a hydrogen atom or an optionally substituted alkyl group, and
R³ is an optionally substituted alkyl group,
excluding a compound wherein both R² and R⁴ are hydrogen atoms and a compound wherein both R¹ and R⁴ are hydrogen atoms, or a tautomer thereof or a salt thereof.

5. The compound according to any one of claims 1 to 4,
wherein
R¹ is an optionally substituted aralkyl group, or a tautomer thereof or a salt thereof.

6. The compound according to any one of claims 1 to 5,
wherein
R² and R⁴ are the same or different and each is a hydrogen atom or a methyl group, excluding a compound wherein both R² and R⁴ are hydrogen atoms and a compound wherein both R¹ and R⁴ are hydrogen atoms, or a tautomer thereof or a salt thereof.

7. The compound according to claim 1 or 3,
wherein
R¹ and R² form, together with the adjacent nitrogen atom, an optionally substituted nitrogen-containing heterocyclic group, or a tautomer thereof or a salt thereof.

8. The compound according to any one of claims 1 to 4,
wherein
R¹ and R² are each a hydrogen atom,
excluding a compound wherein R⁴ is a hydrogen atom, or a tautomer thereof or a salt thereof.

9. A method of producing a compound represented by the formula (1) 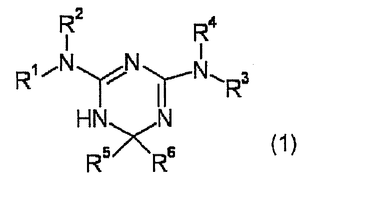 wherein each symbol is as defined in claim 1,
excluding a compound wherein both R² and R⁴ are hydrogen atoms and a compound wherein both R¹ and R⁴ are hydrogen atoms, or a tautomer thereof or a salt thereof, comprising reacting a compound represented by the formula (2) 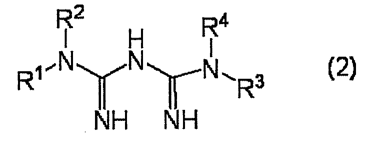 wherein each symbol is as defined in claim 1,
excluding a compound wherein both R² and R⁴ are hydrogen atoms and a compound wherein both R¹ and R⁴ are hydrogen atoms, or a tautomer thereof or a salt thereof, with
a compound represented by the formula (3) 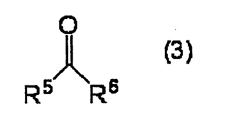 wherein each symbol is as defined in claim 1.

10. A bactericidal/disinfectant agent comprising a compound according to any one of claims 1 to 8, or a tautomer thereof or a salt thereof as an active ingredient.

11. An antiseptic/preservative agent for cosmetics comprising a compound according to any one of claims 1 to 8, or a tautomer thereof or a salt thereof as an active ingredient.
